(19) Europäisches Patentamt
European Patent Office
Office européen des brevets

(11) EP 2 946 725 B1

(12) EUROPEAN PATENT SPECIFICATION

(45) Date of publication and mention
of the grant of the patent:
**29.03.2017 Bulletin 2017/13**

(51) Int Cl.:
***A61B 5/00*** (2006.01)

(21) Application number: **15167716.8**

(22) Date of filing: **14.05.2015**

(54) **PHOTOACOUSTIC APPARATUS**

PHOTOAKUSTISCHE VORRICHTUNG

APPAREIL PHOTOACOUSTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2014 JP 2014100852**

(43) Date of publication of application:
**25.11.2015 Bulletin 2015/48**

(73) Proprietor: **CANON KABUSHIKI KAISHA
Ohta-ku
Tokyo 146-8501 (JP)**

(72) Inventors:
• **BABA, Yoshitaka**
**Ohta-ku, Tokyo (JP)**
• **YAMAMOTO, Hiroshi**
**Ohta-ku, Tokyo (JP)**
• **ABE, Naoto**
**Ohta-ku, Tokyo (JP)**

(74) Representative: **Houle, Timothy James
Canon Europe Ltd
European Patent Department
3 The Square
Stockley Park
Uxbridge, Middlesex UB11 1ET (GB)**

(56) References cited:
**WO-A1-2011/039979    WO-A1-2012/150655
JP-A- 2013 150 745**

**Description**

BACKGROUND OF THE INVENTION

Field of the Invention

The present invention relates to a photoacoustic apparatus that acquires subject information using a photoacoustic effect.

Description of the Related Art

Intensive research is being made on an optical imaging apparatus for use in a medical field. In the optical imaging apparatus, a subject to be inspected (hereinafter referred to simply as a subject) is illuminated with light emitted from a light source such as a laser, and information is obtained based on the incident light and is converted into the form of an image. Photoacoustic imaging (PAI) is known as one of such optical imaging techniques. In the photo acoustic imaging, pulsed light is generated by a light source, and a subject is illuminated with the pulsed light. Energy of the light is absorbed by a tissue of the subject when the light propagates or is scattered in the subject, and correspondingly an acoustic wave (typically, an ultrasonic wave) is generated by the tissue. The generated acoustic wave is received and an image of subject information is generated based on the received signal.

In a state in which a part to be inspected such as a tumor has an optical energy absorption rate different from that of other tissues, when illumination light strikes the part to be inspected, the part to be inspected absorbs energy of the light and instantaneously expands, which causes an elastic wave (photoacoustic wave) to be generated. The generated elastic wave is received by a probe and is mathematically analyzed to obtain information of an inner part of the subject in terms of an initial sound pressure distribution, an absorbed optical energy density distribution, an absorption coefficient distribution, or the like. Based on the obtained information, it is possible to perform a quantitative measurement of a specific substance in the subject, for example, an oxygen saturation of blood, and the like. In recent years, using the above-described photo acoustic imaging, a preclinical study by imaging a blood vessel of a small animal has been intensively performed. Furthermore, a clinical study for applying the photo acoustic imaging to diagnosis of breast cancer or the like has also been intensively performed ("Photoacoustic Tomography: In Vivo Imaging From Organelles to Organs", Lihong V. Wang Song Hu, Science 335, 1458(2012)).

U. S. Patent No. 5713356 discloses a photoacoustic apparatus capable of performing photoacoustic imaging using a probe including an array of acoustic wave reception elements. U. S. Patent No. 5713356 includes a description that a breast tissue is exposed to an electromagnetic wave, and a photoacoustic wave generated in response to the electromagnetic wave is received by a probe. A reception signal output from the probe is stored in a memory. In this technique disclosed in U. S. Patent No. 5713356, an image of a breast tissue is formed using data of the stored reception signal.

In the photoacoustic apparatus such as that disclosed in U. S. Patent No. 5713356, it is necessary to store the reception signal output from the probe as described above. In such a photoacoustic apparatus, there is a need for reducing the amount of data of the stored reception signal.

The photoacoustic apparatus of WO 2011/039979 A1 comprises means to determine an ineffective area, which is an area (e.g. within the subject) for which no information needs to be recorded, and means to stop the activation of an AD converting unit or recording data in a memory in a period in which information is received from the ineffective area. These means are effective to reduce an amount of data.

SUMMARY OF THE INVENTION

The present invention in its first aspect provides a photoacoustic apparatus as specified in claims 1 to 14.
The present invention in its second aspect provides a photoacoustic apparatus as specified in claim 15.
Further features of the present invention will become apparent from the following description of embodiments with reference to the attached drawings.

BRIEF DESCRIPTION OF THE DRAWINGS

Fig. 1 is a diagram illustrating a configuration of a photoacoustic apparatus according to a first embodiment.
Fig. 2 is a flow chart illustrating a measurement process according to the first embodiment.
Figs. 3A to 3D are diagrams illustrating an operation of a photoacoustic apparatus according to the first embodiment.

Fig. 4 is a diagram illustrating a specific example of a configuration of a photoacoustic apparatus according to the first embodiment.

Fig. 5 is a diagram illustrating control signals according to the first embodiment.

Figs. 6A to 6C are diagrams illustrating an operation of a photoacoustic apparatus according to the first embodiment.

Fig. 7 is a diagram illustrating a photoacoustic apparatus according to a second embodiment.

Fig. 8 is a diagram illustrating a photoacoustic apparatus according to a third embodiment.

DESCRIPTION OF THE EMBODIMENTS

Embodiments of the invention are described below with reference to drawings. Each of the embodiments of the present invention described below can be implemented solely or as a combination of a plurality of the embodiments or features thereof where necessary or where the combination of elements or features from individual embodiments in a single embodiment is beneficial. Note that in the embodiments described below, specific dimensions, materials, shapes, and relative positions of elements, parts, units, or the like are described by way of example but not limitation, and an appropriate change or modification is possible depending on a specific configuration, conditions, or the like of an apparatus to which the invention is applied. That is, the invention is not limited to the details of these specific examples unless otherwise stated.

In the embodiments described below, the acoustic wave may be an elastic wave such as a sonic wave, an ultrasonic wave, a photoacoustic wave, a photo-ultrasonic wave, or the like. In the embodiments described below, a photoacoustic apparatus may be an apparatus using a photoacoustic effect configured to illuminate a subject with light (an electromagnetic wave) thereby generating an acoustic wave in the subject, and receive the generated acoustic wave thereby acquiring characteristic information of the inside of the subject.

In the photoacoustic apparatus, the subject information refers to characteristic information of the subject which is in correlation with or represented by an initial sound pressure of an acoustic wave generated by illumination of light, an absorption density or an absorption coefficient of photo energy determined from the initial sound pressure, the concentration of a substance of a tissue, or the like. More specifically, the concentration of a substance is, for example, the concentration of oxyhemoglobin or reduced hemoglobin, or the oxygen saturation. The characteristic information may be acquired in the form of numerical data or in the form of a distribution across positions in a subject, that is, in the form of image data indicating a distribution in terms of an initial sound pressure, an absorption coefficient, an oxygen saturation, or the like.

The present invention may be embodied as a method of receiving an acoustic wave by a photoacoustic apparatus although in the embodiments described below the invention is explained with reference to specific examples of photoacoustic apparatuses. In the drawings used in the following explanation, similar units or elements will be denoted by similar reference numerals or symbols, and a duplicated description thereof will be omitted.

First, a relationship between a light fluence (or radiant exposure or radiant fluence) in a subject and a sound pressure of an acoustic wave generated by a photoacoustic effect is described. The sound pressure $p_0$[Pa] of the acoustic wave generated by the photoacoustic effect is given by formula (1) shown below.

$$p_0 = \mu_a \cdot \Gamma \cdot \Phi \qquad \cdot \cdot \cdot \quad (1)$$

In formula (1), $\mu_a$ denotes an absorption coefficient [1/mm] of a light absorber (a tumor or the like) in a subject. $\Gamma$ denotes a Gruneisen coefficient. $\Phi$ denotes a light fluence [J/mm$^2$] at a position of a light absorber. The light fluence $\Phi$ is also called radiant exposure or radiant fluence. Note that the Gruneisen coefficient $\Gamma$ has a value obtained by dividing the product of the coefficient of volume expansion and the square of the sound speed c by the specific heat. Note that the Gruneisen coefficient $\Gamma$ is substantially constant in a living body.

As may be seen from formula (1), the sound pressure of the generated acoustic wave is proportional to the light fluence. In a case where the subject is an object that strongly scatters light, such as a living body, the light fluence $\Phi$ typically decays exponentially with increasing distance from the position illuminated with light. The decay results in a reduction in the light fluence, which may make it difficult to detect a sound pressure of an acoustic wave generated in some region. An acoustic wave originating from such a region is low in light fluence and is typically low in signal-to-noise ratio. Such an acoustic wave does not likely make a contribution to acquiring high-accuracy subject information of the inside of a subject. Therefore it may be meaningless to store a reception signal of an acoustic wave originating from a region in which the light fluence is low.

In view of the above, in the present embodiment, as described below, the photoacoustic apparatus is configured to estimate a distribution of a light fluence in the subject that will occur when the subject is illuminated with the light for measurement, and extract a position of interest at which the light fluence is lower than a threshold value based on the light fluence distribution. In the measurement, the probe receives a photoacoustic wave generated by the photoacoustic effect and the probe outputs a time-series reception signal in response to the received photoacoustic wave. A particular part of the reception signals corresponding to the position of interest where the light fluence is lower than the threshold value is subjected to the data reduction.

By reducing the amount of data of the reception signal of the photoacoustic wave generated at the position of interest where the light fluence is low, it is possible to reduce the storage capacity necessary to store the data of the reception signal. The data of the reception signal subjected to the data reduction is data that does not make a significant contribution to the acquiring of the high-accuracy subject information, and thus the data reduction does not likely cause a significant reduction in accuracy of the subject information.

The amount of data of the reception signal stored in the memory may be reduced by not storing a particular part of the time-series reception signal, output from the probe, when the particular part is generated at the position of interest where the light fluence is low. Alternatively, the amount of data may be reduced by sampling the reception signal at a lower sampling frequency than the sampling frequency used for the reception signal of the photoacoustic wave generated in a region where the light fluence is high. As described above, there is no particular restriction on the method of reducing the amount of data as long as it is possible to reduce the amount of data of the reception signal of the photoacoustic wave generated at the position of interest where the light fluence is low.

In a case where a region of interest is a two-dimensional region, a position of interest is a pixel. On the other hand, in a case where a region of interest is a three-dimensional region, a position of interest is a voxel.

First Embodiment

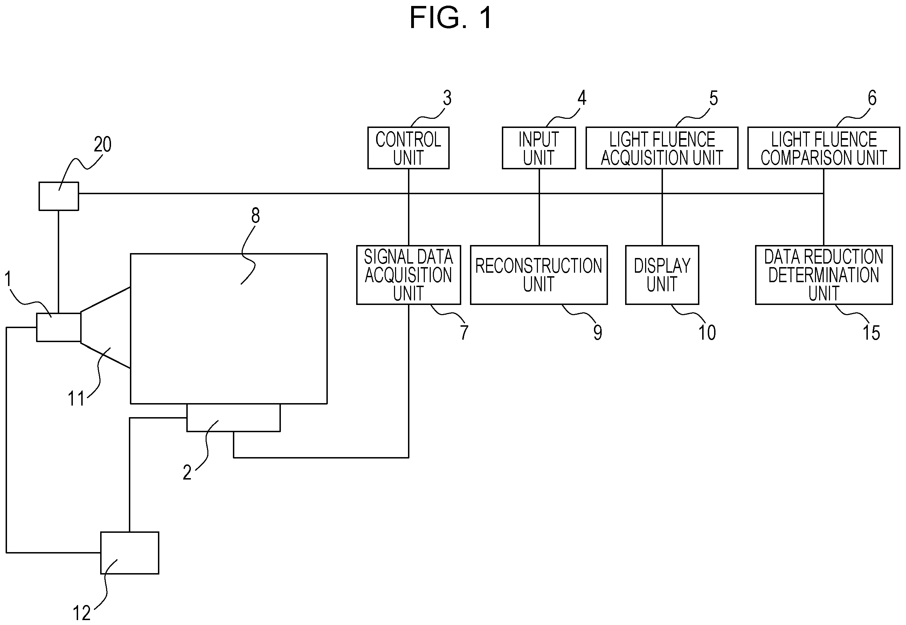

Next, a configuration of a photoacoustic apparatus according to a first embodiment is described below with reference to Fig. 1.

Apparatus Configuration

Fig. 1 is a diagram schematically illustrating a photoacoustic apparatus according to the present embodiment. To acquire information about a subject, the apparatus may be configured as described below.

In the present embodiment, the photoacoustic apparatus includes a light source 20, an illumination unit 1, a probe 2, a control unit 3, an input unit 4, a light fluence acquisition unit 5, a light fluence comparison unit 6, a data reduction determination unit 15, a signal data acquisition unit 7, a reconstruction unit 9, a display unit 10, and a moving mechanism 12.

A detailed description is given below as to each of the units or elements of the photoacoustic apparatus according to the present embodiment.

Light Source 20

The light source 20 according to the present embodiment generates illumination light 11 used to illuminate a subject 8. The light source 20 includes at least one coherent or incoherent pulsed light source. To achieve a photoacoustic effect, a pulse width may be set, for example, to be equal to or smaller than tens of nanoseconds. To perform measurement associated with breast cancer or the like, the illumination light 11 is selected so as to include a particular wavelength that is selectively absorbed by a particular component (for example, hemoglobin) of the living body. The light source 20 may be a laser capable of providing large optical output power. Instead of the laser, a light emitting diode or the like may be used. Examples of lasers usable as the light source 100 include a solid-state laser, a gas laser, a dye laser, a semiconductor laser, and so on.

Illumination Unit 1

The illumination unit 1, which is a part of an optical system, projects illumination light 11 generated by the light source 20 toward the subject 8 such that the subject 8 is illuminated with the illumination light 11 in a manner suitable for the photoacoustic measurement. To achieve a high signal-to-noise ratio for the reception signal, the subject may be illuminated such that a wide surface area or a plurality of different surface areas are illuminated, so as to avoid only a part of a surface of the subject being illuminated. For example, the subject may also be illuminated with light from a side opposite to the probe 2. The illumination unit 1 may include optical elements such as a mirror, a lens that condenses or expands light or changes the shape of the light, or a prism that disperse, refracts, or reflects light, and an optical fiber. Any optical element may be used as long as it is possible to illuminate the subject with light generated by the light source 20 in a specified manner (an illumination direction, an illumination shape, or the like).

Probe 2

The probe 2 includes an acoustic wave reception element configured to receive an acoustic wave generated when the subject 8 is illuminated with light, and to convert the received acoustic wave into a reception signal in the form of an electric signal. The acoustic wave reception element may be an acoustic wave reception element using a piezoelectric phenomenon, an acoustic wave reception element using optical resonance, an acoustic wave reception element using a capacitance change, or the like. Any type of acoustic wave reception element may be used as long as it is capable of receiving an acoustic wave and converting it into an electric signal. In the present embodiment, to receive an acoustic wave at different positions, there may be provided one or more acoustic wave reception elements. One or more acoustic wave reception elements may be arranged in various manners to form the probe 2. For example, the probe 2 may be of a linear type, a 1.5D type, a 2D type, a convex type, an arc type, a hemisphere type, or any other type with an arbitrary shape. Electric signals generated by a plurality of acoustic wave reception elements of the probe 2 are output to the signal data acquisition unit 7.

Control Unit 3

The control unit 3 supplies various parameters to various units or elements with proper timings to control the units or the elements.

Typically, the control unit 3 may be a personal computer (PC) or an electronic circuit board on which circuits such as a CPU, an FPGA, an ASIC, and/or the like are disposed, but the control unit 3 is not limited to such a type. Any type of control unit may be used as long as it is possible to control the photoacoustic apparatus according to the embodiment.

Moving Mechanism 12

The moving mechanism 12 moves the illumination unit 1 and the probe 2 relatively with respect to the subject 8. The moving mechanism 12 includes a driving unit such as a motor or the like and a mechanical part that transmits driving force. The moving mechanism 12 is configured to move the illumination unit 1 and the probe 2 to move the light illumination position and the reception position at which the photoacoustic wave is received according the information associated with the measurement parameter given by the control unit 3. By acquiring reception signal data repeatedly while moving the light illumination position and the reception position at which the photoacoustic wave is received relatively with respect to the subject 8, it is possible to acquire reception signal data necessary to obtain subject information over a large area. The moving mechanism 12 may function as both a probe moving mechanism for moving the probe 2, and an optical system moving mechanism for moving the illumination unit 1 functioning as a part of the optical system. Note that the probe moving mechanism and the optical system moving mechanism may be separately realized.

The moving mechanism 12 outputs position information to the control unit 3 in synchronization with each operation of projecting light by the illumination unit 1 to provide information on the positions of the illumination unit 1 and the probe 2 located when the light illumination is given, that is, when the photoacoustic wave is received.

Input Unit 4

The input unit 4 is a unit used by a user to input particular information to be transferred to the control unit 3. As for the input unit 4, a keyboard, a mouse, a touch panel, a dial, a button, or the like may be used. In a case where a touch panel is used as the input unit 4, the touch panel functioning as the input unit 4 may be combined with the display unit 10.

The input unit 4 is capable of accepting inputting of information as to an optical characteristic value (absorption coefficient, reduced scattering coefficient, or the like) of the subject 8, and other information needed in controlling the photoacoustic apparatus.

Light Fluence Acquisition Unit 5

The light fluence acquisition unit 5 acquires a light fluence distribution of light inside the subject 8 that is to occur when the subject 8 is illuminated with the light. The light fluence distribution may be calculated by a numerical calculation of a transport equation, a numerical calculation of an approximate diffusion equation, a numerical calculation using a Monte Carlo method, or the like. In the calculation of the light fluence distribution, an intensity distribution pattern of the illumination light 11 may be taken into account to improve the accuracy of the calculation. For example, the intensity distribution pattern of the illumination light 11 may be monitored by the illumination unit 1, and the acquired intensity distribution pattern may be input to the light fluence acquisition unit 5.

The light fluence acquisition unit 5 may acquire the light fluence distribution of the illumination light 11 in the subject 8 by predicting the light fluence distribution of the illumination light 11 to be given to the subject 8 based on the illumination condition (the illumination region, the light intensity, the illumination angle, and/or the like) set before the subject 8 is illuminated with the illumination light 11. The light fluence acquisition unit 5 may acquire the light fluence distribution of the actually given illumination light 11 in the subject 8 from the illumination condition of the illumination light 11 actually given to the subject 8. In this case, the light fluence acquisition unit 5 is capable of determining the light fluence distribution in the subject 8 taking into account the result of the measurement of the illumination condition of light illumination actually given to the subject 8.

The light fluence acquisition unit 5 may acquire the light fluence distribution in the subject 8 by setting a boundary condition or a light intensity distribution on the surface of the subject 8 based on the information on the shape of the subject 8. Use of the information on the shape of the subject 8 makes it possible to improve the accuracy of the light fluence distribution of the subject 8. Light Fluence Comparison Unit 6

The light fluence comparison unit 6 refers to the light fluence distribution in the subject 8 acquired by the light fluence acquisition unit 5 and determines whether the light fluence at each position of interest is smaller than the threshold value. The light fluence comparison unit 6 outputs a result of the determination to the data reduction determination unit 15, the reconstruction unit 9, or the like.

Data Reduction Determination Unit 15

The data reduction determination unit 15 determines which part of the time-series reception signal output from the probe 2 is to be, or not to be, subjected to the reduction in the amount of data. That is, the data reduction determination unit 15 determines a period in which the time-series reception signal output from the probe 2 is to be subjected to the reduction in data. The data reduction determination unit 15 outputs a storage control signal specifying whether the time-series reception signal output from the probe 2 is to be subjected to the reduction in data at each reception timing.

Signal Data Acquisition Unit 7

The signal data acquisition unit 7 converts the electric signal output from the probe 2 (the output being based on a detected acoustic wave) to a digital signal and stores the resultant digital signal in a memory in the signal data acquisition unit 7. The signal data acquisition unit 7 receives the storage control signal output from the data reduction determination unit 15, identifies a reception period corresponding to a region in which the light fluence is equal to or greater than the threshold value, samples the reception signal output during this period, and stores the result as reception signal data. The signal data acquisition unit 7 transfers the stored reception signal data to the reconstruction unit 9.

In the present specification, as for the electric signal output by the probe 2 based on the received photoacoustic wave, a signal in the state before being stored in the final-stage memory of the signal data acquisition unit 7 is referred to as the "reception signal", while signal data in the state after being stored in the final-stage memory of the signal data acquisition unit is referred to as the "reception signal data".

The signal data acquisition unit 7 may perform processing on the reception signal output from the probe 2 in terms of a correction of a sensitivity variation among acoustic wave reception elements of the probe 2, an interpolation process to interpolate physically or electrically missing acoustic wave reception elements, or the like. The signal data acquisition unit 7 includes a signal amplification unit that amplifies the analog signal generated by the probe 2, an analog-to-digital converter that converts the analog signal into a digital signal, and the like.

Reconstruction Unit 9

The reconstruction unit 9 functioning as the information acquisition unit acquires subject information at each position of interest in the subject 8 from the reception signal data stored in the signal data acquisition unit 7. That is, the reconstruction unit 9 converts the reception signal data, which is time-series data, into the subject information which is two-dimensional or three-dimensional spatial data.

Furthermore, the reconstruction unit 9 generates a display image such as a tomographic image based on the acquired subject information at each position of interest. The reconstruction unit 9 may perform various correction processes on the acquired subject information in terms of a luminance correction, a distortion correction, a process of identifying and displaying a region of interest, a process of extracting a region of interest, or the like to provide a display image more suitable for use in diagnosis. According to an input given by a user by operating the input unit 4, a parameter used in the acquisition of the subject information may be determined or the display image may be adjusted.

The subject information at each position of interest is obtained by performing a reconstruction process on the reception signal data stored in the signal data acquisition unit 7. The reconstruction process may be, for example, a back projection in a time domain or a Fourier domain widely used in tomographic technology, a phased addition process, or the like. In the case where there is no significant restriction on a maximum allowable processing time, an image reconstruction method based on an inverse problem analysis using an iterative process may be employed. In a case where the probe has a reception focus capability using an acoustic lens or the like, it is possible to acquire the subject information without performing the reconstruction process.

The reconstruction unit 9 transmits the data of the generated display image to the display unit 10.

Display Unit 10

The display unit 10 displays the distribution of the subject information in the subject 8, numerical data associated with a particular region of interest, and/or the like, using the data of the display image received from the reconstruction unit 9 thereby presenting the information to an operator. The distribution of the subject information may be displayed such that the subject information at respective positions of interest in the subject 8 are displayed such that they are arranged one-dimensionally, two-dimensionally, or three-dimensionally.

As for the display unit 10, a liquid crystal display or the like is typically used. Alternatively, a plasma display, an organic EL display, a FED, or other types of displays may be used. The display unit 10 may be provided separately from the photoacoustic apparatus according to the present embodiment.

The functions provided by the light fluence acquisition unit 5, the light fluence comparison unit 6, the data reduction determination unit 15, the signal data acquisition unit 7, and the reconstruction unit 9 may be realized by executing a program on a computer including an operation unit such as a CPU, GPU, or the like. Alternatively, the functions described above may be realized using a circuit such as an FPGA, an ASIC, or the like. Alternatively, the functions described above may be realized by a combination of hardware and a program executed on a computer.

Operation of Photoacoustic Apparatus

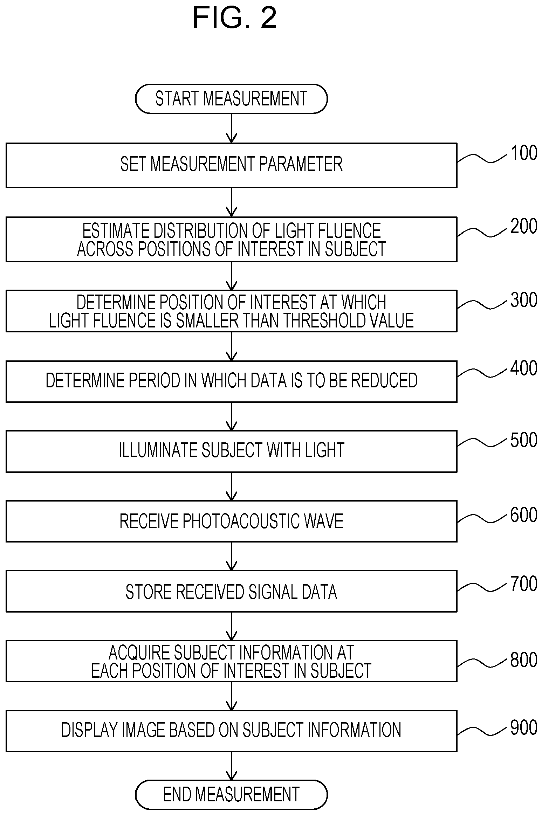

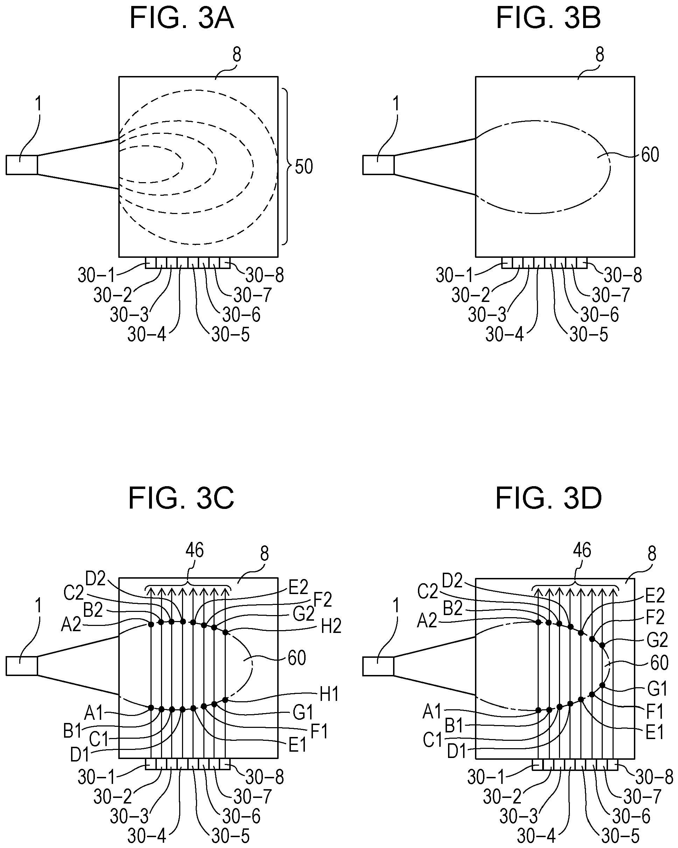

Next, an operation of a photoacoustic apparatus according to the present embodiment is described below with reference to a flow chart illustrated in Fig. 2 in combination with a conceptual diagram in Fig. 3 illustrating the operation of the photoacoustic apparatus.

In step 100, the control unit 3 sets measurement parameters in terms of illumination timing, a light illumination position, a photoacoustic wave reception position, an optical characteristic value (an absorption coefficient, a reduced scattering coefficient, or the like) of the subject 8, a type of a holding unit, and/or the like. That is, the control unit 3 is capable of setting the timing of emitting light from the light source 20, the position of the illumination unit 1 at each light emission timing, and the position of the probe 2 at each light emission timing. The control unit 3 outputs the information about the set measurement parameters to the light source 20, the moving mechanism 12, the light fluence acquisition unit 5, the data reduction determination unit 15, the signal data acquisition unit 7, and/or the like. The control unit 3 may read out measurement parameters stored in advance and may make the setting based on the read-out measurement parameters.

Alternatively, a user may input measurement parameters via the input unit 4, and the control unit 3 may acquire the information about the measurement parameters output from the input unit 4. As for the absorption coefficient and the reduced scattering coefficient, values thereof may be directly input via the input unit 4, or personal information such as an age, a nationality, or the like of the subject 8 may be input via the input unit 4 and the control unit 3 may make setting based on known statistical values corresponding to the input personal information. Alternatively, the measurement parameters may be acquired by another apparatus, and the control unit 3 may acquire the measurement parameters from this apparatus.

In step 200, based on the measurement parameters set by the control unit 3 in step 100, the light fluence acquisition unit 5 acquires a light fluence distribution in the subject 8 in terms of light with which the subject 8 is to be illuminated at each light emission timing.

In the following description, it is assumed by way of example that the setting of the measurement parameters in step 100 is performed so as to obtain a measurement state illustrated in Fig. 3A. The light fluence acquisition unit 5 calculates the light fluence distribution 50 in the subject 8 represented by a dotted line based on a formula such as a light diffusion equation representing light propagation. Alternatively, the light fluence acquisition unit 5 may store in advance a plurality of light fluence distributions corresponding to respective values of the measurement parameters set in step 100, and the light fluence acquisition unit 5 may read a light fluence distribution 50 corresponding to the information about the measurement parameters output from the control unit 3.

In Fig. 3A, the light fluence distribution 50 is represented by dotted isolines. In this example illustrated in Fig. 3A, a maximum light fluence occurs in a region of the subject 8 directly illuminated with the illumination light 11 emitted from the illumination unit 1, and the light fluence decreases with increasing distance from the region illuminated with the illumination light 11.

Examples of the measurement parameters (the illumination condition) used in estimating the light fluence distribution include optical characteristic values of the inside of the subject 8 (for example, an absorption coefficient, reduced scattering coefficient, or the like in the subject 8). In addition, the measurement parameters (the illumination condition) used in estimating the light fluence distribution may include an illumination density distribution of the illumination light 11, a shape (surface coordinates) of the subject 8, a wavelength of the illumination light 11, the total amount of the illumination light 11, a boundary condition depending on a characteristic of a substance in contact with the subject 8. In a case where a holding unit is used to maintain the shape of the subject 8, the shape (surface coordinates) of the holding unit used may be included in the measurement parameters used in estimating the light fluence distribution.

A user may input the spot size of the illumination light 11, the shape of the illumination region, or the like via the input unit 4, and the light fluence acquisition unit 5 may acquire the light fluence distribution in the subject 8 based on the input information. For example, the light fluence acquisition unit 5 may assume that the light fluence is 1 in the inside of a region with the size input by a user extending in an illumination direction and such that the light fluence is 0 in the other regions. This makes it possible to reduce the calculation process of acquiring the light fluence at positions of interest.

In step 300, based on the light fluence distribution in the subject 8 acquired by the light fluence acquisition unit 5 in step 200 for each light emission timing, the light fluence comparison unit 6 determines whether the light fluence is smaller than a threshold value at each position of interest in the subject 8. A determination result at each position of interest is output from the light fluence comparison unit 6 to the data reduction determination unit 15.

Next, a description is given below as to a method of determining, by the light fluence comparison unit 6, whether the light fluence at each position of interest in the subject 8 is smaller than the threshold value.

First, the light fluence comparison unit 6 sets a threshold value individually for each position of interest. In this setting, the light fluence comparison unit 6 may employ values stored in advance for the corresponding threshold values, or may set the threshold values based on values specified by a user via the input unit 4. Alternatively, the light fluence comparison unit 6 may calculate the threshold values based on a light fluence that allows it to generate a minimum sound pressure detectable by the acoustic wave reception element thereby setting the threshold values. Note that the light fluence comparison unit 6 may set an equal value (i.e. the same value) as the threshold values for all positions of interest.

Next, an example is described below as to the method of calculating the threshold values based on a light fluence that allows it to generate a minimum sound pressure detectable by the acoustic wave reception element.

Formula (2) represents an attenuation-compensated noise-equivalent sound pressure value $p_{NEP\_c}(r)$ described below. A threshold value $\Phi_{th}(r)$ of the light fluence at a position of interest represented by a position vector r is represented by formula (3) rewritten from formula (1).

$$p_{NEP\_c}(r) = P_{NEP} \Big/ A(\alpha, |\mathbf{d} - \mathbf{r}|) \qquad \cdots \ (2)$$

$$\Phi_{th}(\mathbf{r}) = p_{NEP\_c}(\mathbf{r}) \Big/ \mu_{a\_BG} \cdot \Gamma \qquad \cdots \ (3)$$

In formula (2), $p_{NEP}$ denotes a noise equivalent sound pressure equal to a noise intensity included in the reception signal divided by a conversion efficiency of a conversion performed by the acoustic wave reception element from a sound pressure to an electric signal. In a case where the electric signal is a voltage signal, the conversion efficiency is represented in units of V/Pa. $A(\alpha, |d - r|)$ denotes a ratio of attenuation of sound pressure that occurs when an acoustic wave propagates between a position of interest at a position vector r and an acoustic wave reception element at a position vector d. The value A is determined by the attenuation coefficient [1/mm] of the subject 8 and the distance |d - r| between the acoustic wave reception element and the position of interest. The attenuation of sound pressure includes attenuation based on a distance-dependent attenuation coefficient $\alpha$, distance-dependent attenuation caused by energy dissipation via spherical wave propagation, cylindrical wave propagation, or the like.

$p_{NEP\_c}(r)$ denotes an attenuation-compensated noise-equivalent sound pressure value obtained by correcting the noise equivalent sound pressure $p_{NEP}$ by dividing the noise equivalent sound pressure $p_{NEP}$ by the attenuation ratio A. When an acoustic wave is generated at a position of interest represented by a position vector r, if it has a sound pressure of $p_{NEP\_c}(r)$, then this acoustic wave is an acoustic wave with a minimum sound pressure detectable by the acoustic wave reception element.

$\mu_{a\_BG}$ denotes an average absorption coefficient of the subject 8. In the photoacoustic imaging, light used has typically a wavelength equal to or smaller than 1000 nm. In this wavelength range, the absorption coefficient of hemoglobin, which is a main component of a blood vessel, is greater than that of water, fat, or the like, which is a main component of a subject such as a living body. Therefore, the absorption coefficient of the blood vessel is greater than the average absorption coefficient $\mu_{a\_BG}$ of the subject 8. Note that, as seen from formula (1), when the absorption coefficient is small, the sound pressure of the generated acoustic wave is small because it is in proportion to the absorption coefficient.

Therefore, when the threshold value is given by $\Phi_{th}(r)$ determined using $p_{NEP\_c}(r)$ and $\mu_{a\_BG}$, if the object to be captured as an image is a blood vessel, it is possible to receive the acoustic wave generated by the blood vessel. In the case where the subject 8 is a living body, the Gruneisen coefficient $\Gamma$ in formula (3) may be given by a user by inputting a typical value of the living body via the input unit 4.

Next, using the threshold values set for the respective positions of interest, the light fluence comparison unit 6 determines whether the light fluence at each position of interest is smaller than the corresponding threshold value. More specifically, the light fluence comparison unit 6 extracts positions of interest at which the light fluence is equal to or greater than the corresponding threshold value and extracts positions of interest at which the light fluence is smaller than the corresponding threshold value. A region surrounded by a dash-dot line in Fig. 3B denotes a region 60 where the light fluence is determined by the light fluence comparison unit 6 as being equal to or greater than the corresponding threshold values. That is, at any position of interest in a region other than the region 60, the light fluence is smaller than the corresponding threshold value. The light fluence comparison unit 6 outputs, to the data reduction determination unit 15, the result of the determination made for each position of interest as to whether the light fluence is smaller than the corresponding threshold value.

In the present embodiment, the threshold values for respective positions of interest are equally set to a certain value, and a region in which the light fluence is equal to or greater than the threshold value is determined as the region 60, which denoted by being surrounded by an isoline in Fig. 3B. The reception signal of the photoacoustic wave generated in the region 60 determined in the above-described manner is a reception signal that makes a large contribution to acquiring high-accuracy subject information. On the other hand, the reception signal of the photoacoustic wave generated in the region other than the region 60 does not make a large contribution to acquiring high-accuracy subject information, and thus it is advantageous to reduce the amount of data thereof.

The light fluence comparison unit 6 may store results of determinations made under a plurality of illumination conditions for respective regions of interest as to whether the light fluence is smaller than the threshold value, and the light fluence comparison unit 6 may read out a determination result corresponding to an illumination condition set in step 100. In this case, the light fluence comparison unit 6 may output the read-out determination result to the data reduction determination unit 15. Note that the measurement parameters that determine the illumination condition are measurement parameters that are allowed to be used in estimating the light fluence distribution described above in step 200.

In step 400, based on the information of the determination made in step 300 as to the positions of interest where the light fluence is smaller than the threshold value, the data reduction determination unit 15 determines a particular part of the time-series reception signal output in step 600 (described later) that is not to be stored in step 700 (described later). That is, the data reduction determination unit 15 determines a period in which the amount of data of the reception signal is reduced in step 700, that is, a period in which the reception signal is not stored in step 700. The data reduction determination unit 15 then generates a storage control signal that controls whether the reception signal is stored or not at each sampling timing of the time-series reception signal, and the data reduction determination unit 15 outputs the storage control signal to the signal data acquisition unit 7. Hereinafter, the period in which the amount of data of the reception signal data is reduced is denoted by a "data reduction period", and a physical region corresponding to this period is denoted by a "data reduction region".

For example, the data reduction determination unit 15 determines an intersection between the region 60 and a normal vector 46 of each acoustic wave reception element 30-1 to 30-8 normal to the reception plane thereof as illustrated in Fig. 3C.

Subsequently, the data reduction determination unit 15 calculates the distance between the acoustic wave reception element 30-1 and the intersection A1. Furthermore, the data reduction determination unit 15 calculates the distance between the intersection A1 and the intersection A2. The locations of the respective acoustic wave reception elements 30-1 to 30-8 are known in advance, and thus information of the locations of the respective acoustic wave reception elements 30-1 to 30-8 may be stored in advance. The data reduction determination unit 15 may read out the stored information of the locations of the respective acoustic wave reception elements 30-1 to 30-8 and may use the information in calculating the distances.

Subsequently, the data reduction determination unit 15 generates a storage control signal specifying not to store the reception signal output in the period corresponding to the region between the acoustic wave reception element 30-1 and the intersection A1. Furthermore, the data reduction determination unit 15 generates a storage control specifying to store the reception signal output in the period corresponding to the region between the intersection A1 and the intersection A2. For example, the data reduction determination unit 15 may calculate the period in which the reception signal is not to be stored by dividing the distance between the acoustic wave reception element 30-1 and intersection A1 by the sound speed in the subject 8. Furthermore, the data reduction determination unit 15 calculates the period by dividing the distance between the intersection A1 and the intersection A2 by the sound speed in the subject 8. Thereafter, the data reduction determination unit 15 sets the period, in which the reception signal corresponding to the distance between the intersection A1 and the intersection A2 is to be stored, to follow the period in which the reception signal corresponding to the region between the acoustic wave reception element 30-1 and the intersection A1 is not to be stored. Furthermore, the data reduction determination unit 15 generates a storage control signal to control the reception signal not to be stored in a period following the period in which the reception signal corresponding to the region between the intersection A1 and the intersection A2 is stored.

The sound speed in the subject 8 may be specified by a user via the input unit 4, or may be set based on a value stored in advance. In a case where a material such as an acoustic matching material is disposed between the acoustic wave reception element and the subject, the storage control signals may be generated taking into account the sound speed in this material.

The data reduction determination unit 15 generates storage control signals also for the other acoustic wave reception elements.

In step 500, the moving mechanism 12 moves the illumination unit 1 and the probe 2 to the positions set by the control unit 3 in step 100. Subsequently, the light source 20 generates light according to the illumination timing set by the control unit 3. The illumination unit 1 directs the light generated by the light source 20 toward the subject 8 such that the subject 8 is illuminated with the light. A synchronization signal may be generated to indicate that the light is emitted from the light source 20. There may be provided a detection unit configured to detect that the light is emitted from the illumination unit 1, and a light emission detection signal may be output from the detection unit. For example, a photodetector or the like may be used as the detection unit.

In step 600, the acoustic wave reception element 30 disposed on the probe 2 receives the photoacoustic wave generated in the subject 8 in response to the light illumination in step 500, and the acoustic wave reception element 30 outputs a time-series reception signal.

In step 700, the signal data acquisition unit 7 stores the time-series reception signal, output in step 600 from the acoustic wave reception element 30, in accordance with the storage control signal generated in step 400.

That is, the signal data acquisition unit 7 does not store the reception signal output in the period corresponding to the distance between the acoustic wave reception element 30-1 and the intersection A1, while the signal data acquisition unit 7 stores the reception signal output in the period corresponding to the distance between the intersection A1 and the intersection A2. As for the reception signal output after the period corresponding to the distance between the intersection A1 and the intersection A2, the signal data acquisition unit 7 does not store it. Storing the reception signal may be started in response to the light illumination timing given in step 500.

In step 800, the reconstruction unit 9 acquires subject information associated with each position of interest in the subject 8 based on the reception signal data stored in step 700 in the signal data acquisition unit 7. The reconstruction unit 9 may acquire the subject information for only positions of interest in the region 60 in which the light fluence is determined in step 300 as being greater than the threshold value. This makes it possible to selectively acquire the subject information associated with the region in which the light fluence is high and thus the generated sound pressure is estimated to be high. This allows it to reduce the amount of processing necessary to acquire the subject information. Note that because the subject information is acquired for the region in which the generated sound pressure is estimated to be high, the acquired subject information is high in accuracy.

In step 900, the reconstruction unit 9 generates display image data of the subject information based on the subject information acquired in step 800, and displays the display image data of the subject information on the display unit 10.

As described above, in the present embodiment, the photoacoustic apparatus is capable of changing the period in which the amount of data of the time-series reception signal output from each acoustic wave reception element 30 is reduced, depending on the light fluence at the position of interest, such that the amount of data is reduced selectively for the amount of data of the reception signal generated in the region in which the light fluence is smaller than the threshold value. That is, it is possible to selectively acquire the data of the reception signal of the photoacoustic wave generated in the region in which the light fluence is equal to or greater than the threshold value. Thus, it is possible to reduce the amount of data for the reception signal of the photoacoustic wave that makes no significant contribution to the acquisition of high-accuracy subject information, because that is generated in the region in which the light fluence is low, thereby reducing the memory capacity necessary to store the data while accurately acquiring the subject information in the region in which the light fluence is high.

In the present embodiment, as described above, the amount of data of the reception signal stored in the signal data acquisition unit 7 is reduced by not storing the reception signal of the photoacoustic wave generated in the region in which the light fluence is smaller than the threshold value. The signal data acquisition unit 7 may sample the reception signal at a lower sampling frequency in the data reduction period than the sampling frequency used for the other reception signals. This also makes it possible to relatively reduce the amount of data of the reception signal of the photoacoustic wave generated in the region in which the light fluence is smaller than the threshold value. In the case where the amount of data is reduced by reducing the sampling frequency, the subject information associated with the region in which the light fluence is lower than the threshold value is acquired from the reception signal data although a reduction in the resolution occurs. Therefore, in this case, it is possible to get information about the region in which the light fluence is smaller than the threshold value while achieving the reduction in the amount of data stored in the memory.

Note that there is no particular restriction on the method of reducing the amount of data as long as the method allows it to reduce the amount of data of the reception signal of the photoacoustic wave received in the data reduction period.

In the case where the measurement parameters are set in step 100 such that illumination with the illumination light 11 is performed at a plurality of timings, the process from step 200 to step 700 is performed for each timing.

Also in a case where the locations of the acoustic wave reception elements 30 are different, as illustrated in Fig. 3D, from those in Fig. 3C, the process from step 200 to step 700 is performed repeatedly. In the case where the illuminating with the illumination light 11 is performed under the same illumination condition at a plurality of timings as with cases illustrated in Fig. 3C and Fig. 3D, steps 200 and 300 may be skipped. That is, the result of the determination obtained in step 300 at a previous timing as to whether the light fluence at each position of interest is smaller than the threshold value or not may be used in steps 400 to 700 at a following timing.

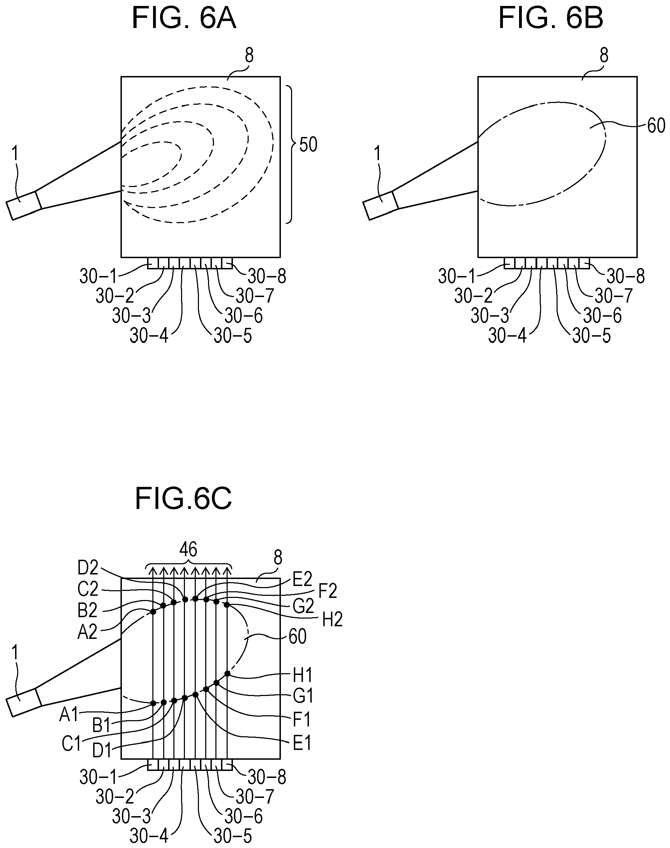

On the other hand, in a case where the subject 8 is illuminated, as illustrated in Fig. 6A, with the illumination light 11 under an illumination condition different from the illumination condition used in Fig. 3A, it is necessary to perform steps 200 to 400. In step 300, the light fluence acquisition unit 5 acquires a light fluence distribution 50 in the subject 8 as illustrated in Fig. 6A. Subsequently, in step 400, based on the light fluence distribution 50 illustrated in Fig. 6A, the light fluence comparison unit 6 determines the region 60 in which the light fluence is equal to or greater than the threshold value as illustrated in Fig. 6B. The light fluence comparison unit 6 outputs, to the data reduction determination unit 15, a result of the determination for the case in Fig. 6C as to whether the light fluence at each position of interest is smaller than the threshold value. As may be seen from the comparison between Fig. 3B and 6B, in a case where there is a difference in the illumination condition associated with the illumination light 11, the result of the determination may be different as to whether the light fluence at each position of interest is smaller than the threshold value.

The subject information may be acquired in step 800 each time the reception signal data at a particular timing is acquired, and, when the acquisition of the subject information is complete for all timings, the subject information acquired at respective timings may be integrated into complete subject information. Alternatively, after the acquisition of the reception signal data is complete for all timings, the subject information may be acquired in step 800 based on all reception signal data.

In the above description, it is assumed by way of example that measurement parameters are changed from one timing to another. Alternatively, reception signal data may be acquired using the same measurement parameters for a plurality of timings. This makes it possible to increase the signal-to-noise ratio for the acquired reception signal data, which makes it possible to acquire high-accuracy subject information.

In the present embodiment described above, the light fluence distribution is acquired based on measurement parameters before illumination light is provided, and the storage control signal is generated based on the light fluence distribution. Alternatively, the storage control signal may be generated after the illumination light is provided. That is, steps 200 to 400 may be performed after step 500, and step 600 may be performed after step 400. In this case, a photodetector may detect, in step 500, the illumination light 11 emerging from the subject 8 after passing through the subject 8, and the light fluence acquisition unit 5 may acquire the light fluence distribution in the subject 8 from the detection signal of the illumination light 11 using a light diffusion tomography technique, or the like. This method makes it possible to acquire the light fluence distribution based on the illumination light 11 actually striking the subject 8. Specific Example of Reduction in Amount of Data

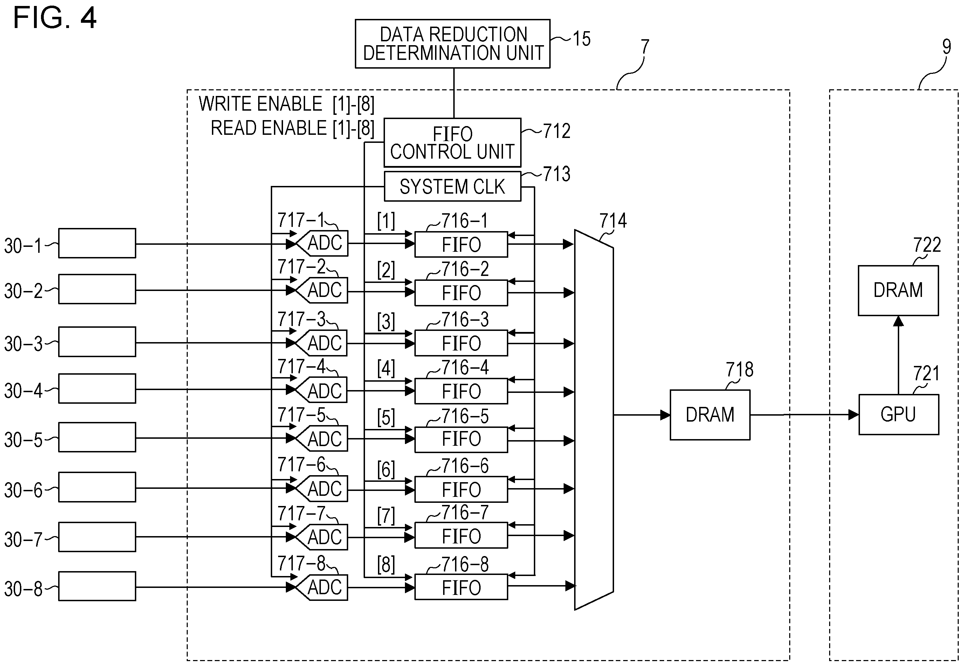

Referring to the signal data acquisition unit 7 illustrated in Fig. 4, a specific example is described below as to a method of reducing the amount of data in step 700 by the signal data acquisition unit 7 according to the storage control signal generated in step 400 by the data reduction determination unit 15.

The acoustic wave reception elements 30-1 to 30-8 respectively convert received photoacoustic waves into electric signals and output the resultant electric signals to corresponding analog-to-digital converters (ADC) 717-1 to 717-8. The ADCs 717-1 to 717-8 respectively sample the electric signals at a frequency according to a clock signal output from a system clock (CLK) 713 and convert the electric signals to digital signals. The resultant digital signals are output to first in, first out (FIFO) memories 716-1 to 716-8. The FIFO memories 716-1 to 716-8 store the respective digital signals output from the ADCs 717-1 to 717-8 according to the clock signal output from the system clock 713 and enable signals output from a FIFO control unit 712.

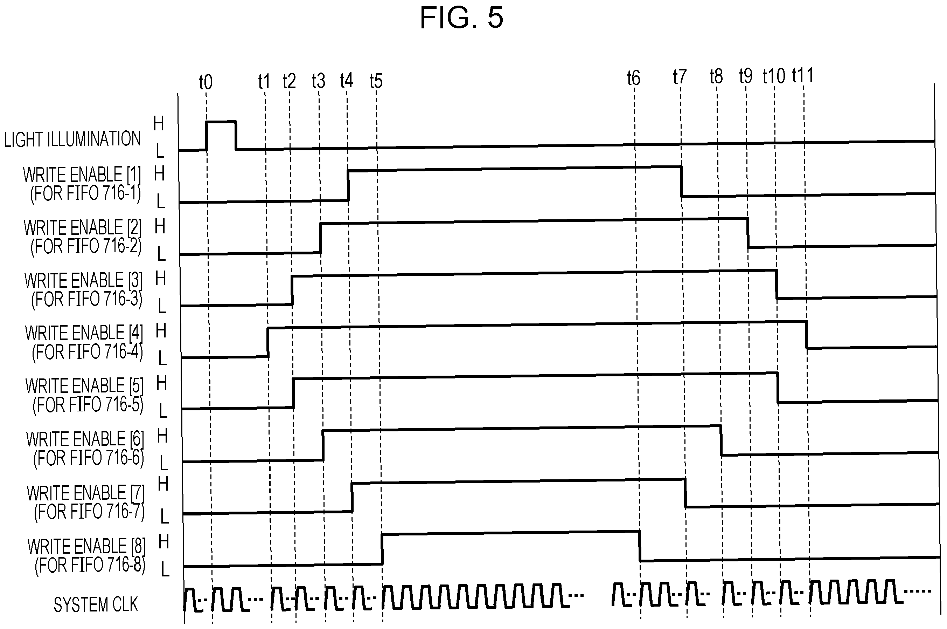

In this process, the FIFO control unit 712 adjusts the timing of outputting write enable signals, based on the information output from the data reduction determination unit 15, as to the periods during which the amount of data is to be reduced for the reception signals output from the respective acoustic wave reception elements 30-1 to 30-8. More specifically, for example, the timings of outputting the respective write enable signals [1] to [8] to the FIFO memories 716-1 to 716-8 are adjusted as illustrated in Fig. 5. In Fig. 5, when the write enable signal is at an L level for some of the FIFO memories 716-1 to 716-8, then writing to this FIFO memory is not performed. On the other hand, when the write enable signal is at an H level for some of the FIFO memories 716-1 to 716-8, then writing to this FIFO memory is performed. Note that in Fig. 5, it is assumed that illumination light is provided at time t0.

For the FIFO memory 716-1 associated with the acoustic wave reception element 30-1, data reduction is not performed in a period from t4 to t7. This period from t4 to t7 corresponds to the region from the intersection A1 and the intersection A2 in Fig. 3C. In this period from t4 to t7, the acoustic wave reception element 30-1 receives the photoacoustic wave generated in the region 60 in which the light fluence is equal to or greater than the threshold value, and thus the write enable signal [1] is set to the H level, and the digital signal is stored in the FIFO memory 716-1.

On the other hand, a period from t0 to t4 and a period after t7 correspond to periods in which the amount of data is reduced. That is, these periods, in which the amount of data is reduced, correspond to the region between the acoustic wave reception element 30-1 and the intersection A1 in Fig. 3C and the region extending from the intersection A2 in a direction away from the acoustic wave reception element 30-1. In these periods, the acoustic wave reception element 30-1 receives the photoacoustic wave generated in the region, other than region 60 in Fig. 3C, in which the light fluence is smaller than the threshold value, and thus the write enable signal [1] is set to the L level, and the digital signal is not stored in the FIFO memory 716-1.

The write enable signals associated with the other FIFO memories 716-2 to 716-8 are individually controlled in a similar manner such that the data is reduced for the signals received in the respective data reduction periods for the FIFO memories 716-1 to 716-8.

The FIFO memories 716-1 to 716-8 transfer the stored digital signals to a DRAM 718 which is a final-stage memory in accordance with the clock signal output from the system clock 713 and the enable signals output from the FIFO control unit 712. More specifically, a select switch 714 selects one of the FIFO memories 716-1 to 716-8 such that the selected FIFO memory is connected to the DRAM 718 thereby allowing it to transfer the digital signal from the selected FIFO memory to the DRAM 718. Thus, the reception signal in any data reduction period is stored in the DRAM 718 in the form of a digital signal after the amount of data thereof is reduced. In the storing of data in the DRAM 718, the reception signal in the data reduction period is reduced, and thus a reduction in amount of data is achieved. Thus, in the present embodiment, the DRAM 718 does not need to have as much memory capacity as would be required to store as all time-series reception signals, and thus it is possible to reduce the memory capacity of the DRAM 718. Note that the DRAMs 718 and 722 may be replaced with other types of storage medium such as SRAMs, flash memories, or the like. That is, there is no particular restriction on the type of the storage medium used as the DRAMs 718 and 722 as long as a storage capacity necessary in the system operation and necessary writing and reading speeds are satisfied.

In the present specification, the reception signal data refers to time-series signal data being immediately before being used in acquiring the subject information by the reconstruction unit 9 described later. That is, the reception signal data refers to time-series signal data stored in the final-stage memory of the signal data acquisition unit 7. Therefore, in the present embodiment, it is sufficient if it is possible to reduce the amount of data stored in the final-stage memory of the signal data acquisition unit 7.

Thus, for example, data reduction may be performed such that when data is stored in a first-stage memory, reduction in amount of data is not performed, but the amount of data is reduced when the data is transferred from a previous-stage memory to a following-stage memory by reducing the amount of data of the reception signal corresponding to the data reduction period, thereby achieving the reduction in amount of data of the data stored in the final-stage memory.

To reduce the necessary memory capacity of each memory in the signal data acquisition unit 7, the amount of data may be reduced at as earlier a stage as possible. More specifically, the amount of data may be reduced for the data stored in the first-stage memory, that is, the FIFO memory 716, in the signal data acquisition unit 7 as in the present embodiment. By performing the data reduction when the data is stored in the first-stage memory, it is possible to reduce the amount of data transferred to parts following the first-stage memory, and thus it is possible to reduce a time necessary to transfer the data. As a result, it is possible to increase the speed of the process from the step of receiving the photoacoustic wave to the step of acquiring the subject information.

Note that the transfer destination to which the digital signal acquired by the first-stage memory is transferred is not limited to the following-stage memory. For example, the digital signal acquired by the first-stage memory may be output to an operation unit, which in turn may perform pre-processing such as a noise handling process, or the like, and then may transfer the resultant signal to the following-stage memory.

The reception signal data may be stored in relation to information indicating a location of a supporting unit, information indicating the number of times light emission is performed, and/or the like. For example, when the digital signal is transferred from the FIFO memories 716-1 to 716-8 to the DRAM 718, a header or a trailer may be attached at the beginning or the end of a set of digital signals. The header or the trailer may include information on the number identifying the reception element by which the set of digital signals is acquired, the information indicating the position of the supporting unit, the number of times light emission is performed, the data reduction period, and/or the like. Both the header and the trailer may be provided or only one of them may be provided. In the case where both the header and the trailer are provided, a determination may be made appropriately as to what information is described in which data field.

In the present embodiment, the data of the photoacoustic wave is reduced by controlling whether the writing to the FIFO memory is enabled or disabled depending on whether the data of the photoacoustic wave originates from the data reduction region or not. However, the method of reducing the data is not limited to this method. For example, the system clock 713 may be supplied to the ADCs 717-1 to 717-8 only when the photoacoustic wave originating from the subject is received. In this case, the system clock 713 is always supplied to the FIFO memories 716-1 to 716-8. Alternatively, the system clock 713 may be supplied to the FIFO memories 716-1 to 716-8 only when the photoacoustic wave originating from the region 60 is received. In this case, the system clock 713 is always supplied to the ADCs 717-1 to 717-8. Alternatively, the system clock 713 may be supplied to the ADCs 717-1 to 717-8 and the FIFO memories 716-1 to 716-8 only when the photoacoustic wave originating from the region 60 is received.

In the case where the sampling frequency is reduced in the data reduction period, the clock signals from the system clock 713 are individually supplied to the ADC 717-N and the FIFO memory 716-N (N = 1 to 8) such that the clock signals function as the sampling clock signal and the write clock signal (not illustrated). The write enable signals associated with the respective FIFO memories 716 are all set to the H level at the same time with reference to the light illumination timing. The frequency of the clock signal supplied from the system clock 713 is reduced for the ADCs 717 and FIFO memories 716 connected to the acoustic wave reception elements 30 that are in the middle of receiving the acoustic wave in the data reduction period. When the acoustic wave reception elements 30 are in the middle of receiving the acoustic wave in periods other than the data reduction period, the frequency of the clock signal is increased which is supplied from the system clock 713 to the ADCs 717 and FIFO memories 716 connected to the acoustic wave reception elements 30.

Even when the same clock signal is supplied from the system clock 713 to all ADCs 717 and FIFO memories 716, it is possible to effectively reduce the sampling frequency in the data reduction period as described below. For example, when the sampling clock signal with the same frequency $f_H$ is supplied to all ADCs 717 and FIFO memories 716, the write enable signal is turned to the H level for one cycle every K clock cycles for those applied to the FIFO memories 716 connected to the acoustic wave reception elements 30 in the middle of receiving the acoustic wave in the data reduction period thereby effectively setting the sampling frequency to $f_H/K$. In this way, it is possible to reduce the sampling frequency in the data reduction period individually for each of all FIFO memories 716. Any other methods may be employed as long as it is possible to selectively reduce the amount of data of the reception signal of the photoacoustic wave generated in the region in which the light fluence is smaller than the threshold value.

Second Embodiment

In the first embodiment described above, based on the normal vectors of the acoustic wave reception elements, the determination is made as to which reception signal is to be subjected to the reduction in the amount of data. In a second embodiment described below, the determination as to which reception signal is to be subjected to the reduction in the amount of data is made based on directivity angles of the acoustic wave reception elements. In the present embodiment, units, elements, and steps similar to those in the first embodiment described above are denoted by similar reference numerals or symbols, and a further detailed description thereof is omitted.

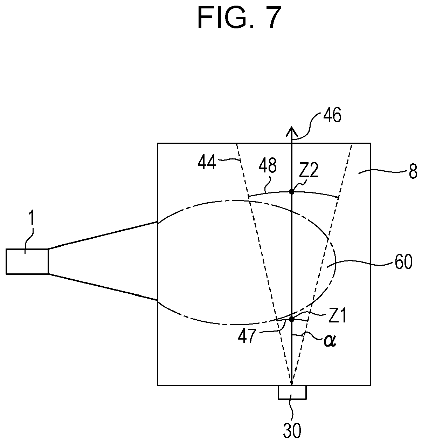

In Fig. 7, a dash-dot line denotes a region 60 where the light fluence is equal to or greater than the threshold value, and a broken line denotes a region 44 in which the directivity angle of the acoustic wave reception element 30 is within a directivity angle $\alpha$. Typically, the acoustic wave reception element 30 has a highest reception sensitivity to an acoustic wave incident from the direction normal to the reception plane, and the reception sensitivity decreases with increasing incident angle. In the present specification, the maximum reception sensitivity is denoted by S, and a directivity angle $\alpha$ is defined as a particular incident angle at which the reception sensitivity is equal to S/2, that is, equal to one-half the maximum reception sensitivity.

That is, the acoustic wave reception element 30 is capable of receiving, with high sensitivity, the photoacoustic wave generated in the region 44 that is within the directivity angle $\alpha$. Therefore, the reception signal of the photoacoustic wave generated in the region 44 within the directivity angle $\alpha$ is worth storing.

For example, the region 60 does not include a point Z1 on a normal vector 46 normal to the reception plane of the acoustic wave reception element 30. On the other hand, there is an overlap between the region 60 and an equidistant arc 47 whose distance from the acoustic wave reception element 30 is equal to the distance from the acoustic wave reception element 30 and the point Z1. The arc 47 is included in the region 44 that is within the directivity angle $\alpha$. That is, a reception signal corresponding to the distance between the acoustic wave reception element 30 and the point Z1 includes a reception signal generated by receiving with high sensitivity a photoacoustic wave generated in the region 60. The reception signal corresponding to the distance between the acoustic wave reception element 30 and the point Z1 refers to a particular part of time-series reception signal output from the acoustic wave reception element, obtained at a reception timing equal to the distance between the acoustic wave reception element 30 and the point Z1 divided by the sound speed in the subject 8.

Similarly, a point Z2 on a normal vector 46 normal to the reception plane of the acoustic wave reception element 30 is not included in the region 60, but an equidistant arc 48 whose distance from the acoustic wave reception element 30 is equal to the distance between the acoustic wave reception element 30 and the point Z2 has an overlap with the region 60. That is, a reception signal corresponding to the distance between the acoustic wave reception element 30 and the point Z2 includes a reception signal output based on a photoacoustic wave generated in the region 60 and received with high sensitivity.

Thus, in view of the above, the data reduction determination unit 15 generates a storage control signal specifying not to store the reception signal output in the period corresponding to the region between the acoustic wave reception element 30 and the point Z1. Furthermore, the data reduction determination unit 15 generates a storage control signal specifying to store the reception signal output in the period corresponding to the region between the point Z1 and point Z2. The data reduction determination unit 15 generates a storage control signal specifying not to store the reception signal output after the period in which the reception signal corresponding to the region between the point Z1 and the point Z2 is stored.

By determining the reception signal to be subjected to the reduction in amount of data in the above-described manner, it is possible to selectively store the reception signal of the photoacoustic wave generated in the region within the range of the directivity angle in which the light fluence is equal to or greater than the threshold value.

Third Embodiment

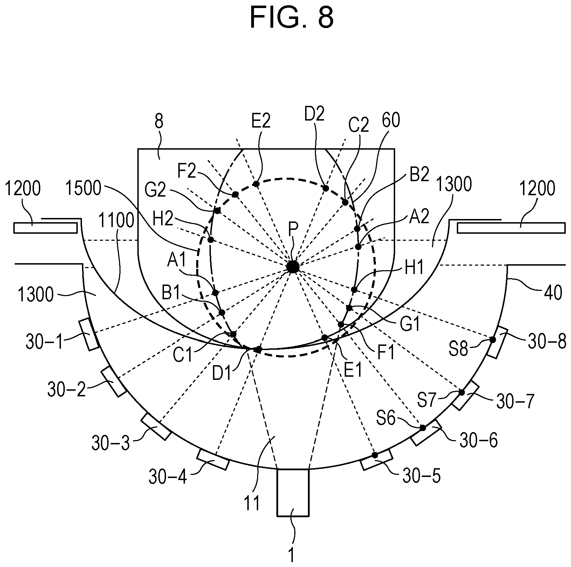

Next, a photoacoustic apparatus according to a third embodiment is described below with reference to Fig. 8. Fig. 8 is a schematic diagram illustrating the photoacoustic apparatus according to the third embodiment. In the present embodiment, units, elements, and steps similar to those in the first embodiment described above are denoted by similar reference numerals or symbols, and a further detailed description thereof is omitted.

A probe, used in the present embodiment, is configured such that a plurality of acoustic wave reception elements 30 are disposed on a hemispherical surface of a supporting unit 40 with a hemispherical shape as illustrated in Fig. 8. Fig. 8 illustrates a cross section taken along a center axis of the hemisphere of the supporting unit 40 with the hemispherical shape.

In a case where subject information is acquired using a reception signal obtained by receiving an acoustic wave when the probe is at a particular position, a highest resolution is obtained typically at a point where highest-sensitivity axes of acoustic wave reception elements converge. In the present specification, an axis in a direction in which the highest reception sensitivity is obtained is referred to as a directional axis. The resolution of the subject information decreases with increasing distance from the point where the directional axes converge. As for photoacoustic waves generated in a region inside a boundary at which the resolution is equal to one-half the highest resolution, it is allowed to expect that each acoustic wave reception element disposed on the supporting unit is capable of receiving the photoacoustic waves with high sensitivity. Thus, in the present embodiment, the region in which the resolution is one-half the highest resolution is referred to as a high sensitivity region.

In general, each acoustic wave reception element 30 has a highest reception sensitivity in a direction normal to its reception plane (surface). In the present embodiment, the plurality acoustic wave reception elements 30 are disposed such that the directional axes of the plurality of acoustic wave reception elements 30 converge substantially at a center of curvature P of the hemispherical shape, thereby forming a high sensitivity region 1500 centered at the center of curvature P as represented by a broken line in Fig. 8.

For example, the high sensitivity region 1500 may be set as a region with a substantially spherical shape centered at a center of curvature P at which a highest resolution $R_H$ is obtained and having a radius r given by formula (4).

$$r = \frac{r_0}{\phi_d} \sqrt{R^2 - R_H{}^2} \qquad (4)$$

In formula (4), R denotes a lower limit resolution in the high sensitivity region 1500, $R_H$ denotes the highest resolution, $r_0$ denotes a radius of the supporting unit 40 with the hemispherical shape, and $\Phi_d$ denotes a diameter of the acoustic wave reception element 30. For example, R is set to be equal to one-half the highest resolution obtained at the center of curvature P as described above.

In the present embodiment, there is no particular restriction on the arrangement of the plurality of acoustic wave reception elements 30, and the acoustic wave reception elements 30 do not necessarily need to be disposed on the hemispherical shape as illustrated in Fig. 8. The plurality of acoustic wave reception elements 30 may be disposed at arbitrary locations as long as the directional axes thereof converge within a particular region and a high sensitivity region is formed. That is, the plurality of acoustic wave reception elements may be disposed on a curved surface shape such that the high sensitivity region 1500 is formed. In the present specification, the "curved surface" may be a spherical surface having a spherical shape or a hemispherical shape with an opening. Even when a surface has an unevenness, if the unevenness is sufficiently small, then the surface may be regarded as a curved surface. An ellipsoidal body (a shape with a surface of a quadratic surface obtained by three-dimensionally expanding an ellipse) may be regarded as a spherical surface if the shape is sufficiently close to a spherical surface.

In a case where a plurality of acoustic wave reception elements are disposed along a supporting unit with a shape obtained by cutting a sphere at an arbitrary cross section, the directional axes converge at the center of curvature of the shape of the supporting unit. The supporting unit 40 with the hemispherical shape according to the present embodiment is an example of a supporting unit with a shape obtained by cutting a sphere at an arbitrary cross section. In the present specification, the shape obtained by cutting a sphere at an arbitrary cross section is referred to as a sphere-based shape. Note that in a case where a plurality of acoustic wave reception elements supported by the supporting unit with a sphere-based shape, the plurality of acoustic wave reception elements are supported on the spherical surface.

As described above, each acoustic wave reception element 30 is capable of receiving a photoacoustic wave generated at the center of curvature P with high sensitivity. Furthermore, each acoustic wave reception element 30 is also capable of receiving a photoacoustic wave generated in the high sensitivity region 1500 with high sensitivity. That is, the particular part of the time-series reception signal output from the acoustic wave reception element that corresponds to the center of curvature P or the high sensitivity region 1500 is a reception signal that makes a significant contribution to acquisition of accurate subject information. As described above in the first embodiment, a reception signal of a photoacoustic wave generated in a region 60 in which the light fluence is equal to or greater than a threshold value is also a reception signal that makes a significant contribution to acquisition of accurate subject information.

In the photoacoustic apparatus according to the present embodiment, in view of the above, the acquisition of data of a reception signal is performed so as to selectively acquire a reception signal of a photoacoustic wave generated in a region that is an intersection between the center of curvature P, or the high sensitivity region 1500, and the region 60 in which the light fluence is equal to or greater than the threshold value. That is, in the present embodiment, the signal data acquisition unit 7 may generate reception signal data and store it such that the amount of data is reduced for the reception signal of the photoacoustic wave generated at any position of interest at which the light fluence is smaller than the threshold value and which is different from the center of curvature P. Furthermore, in the present embodiment, the signal data acquisition unit 7 may generate reception signal data and store it such that the amount of data is reduced for the reception signal of the photoacoustic wave generated at any position of interest at which the light fluence is smaller than the threshold value, and which is not included in the high sensitivity region 1500.

In the present embodiment, it is assumed by way of example that the subject 8 is a breast, and the subject 8 is held by the shape holding unit 1100 to maintain the shape of the subject 8. The shape holding unit 1100 is attached to an attaching unit 1200. In a case where a plurality of shape holding units used to hold a subject E in a plurality of shapes, the attaching unit 1200 may be configured to be capable of supporting a plurality of shape holding units. In other words, a plurality of holding units 1100 may be provided, where each of the plurality of holding units 1100 has a different shape such that each of the holding units 1100 is capable of holding the shape of a differently shaped subject 8. Thus, a plurality of differently shaped subjects 8 can be accommodated by selection of an appropriately shaped holding unit 1100 from a plurality holding units 1100.

The illumination unit 1 functioning as an exit opening of the illumination light 11 is provided in a bottom part (lower pole) of the hemisphere of the supporting unit 40 such that when the moving mechanism 12 moves the supporting unit 40, the illumination unit 1 and the plurality of acoustic wave reception elements 30 also move synchronously. In this structure, the moving mechanism 12 functions as both a probe moving mechanism that moves the supporting unit 40 and an optical system moving mechanism that moves the illumination unit 1 which is a part of the optical system.

An acoustic matching material 1300 is disposed between the subject 8 and the shape holding unit 1100 to acoustically couple the subject 8 to the acoustic wave reception elements 30. An acoustic matching material 1300 is also disposed between the acoustic wave reception elements 30 and the shape holding unit 1100 to acoustically couple the acoustic wave reception elements 30 to the shape holding unit 1100. Note that the acoustic matching material 1300 between the subject 8 and the shape holding unit 1100 may be different from the acoustic matching material 1300 between the acoustic wave reception element 30 and the shape holding unit 1100.

It may be desirable that the acoustic matching material 1300 has an acoustic impedance close to the acoustic impedance of subject 8 and close to the acoustic impedance of the acoustic wave reception elements 30. It may be more desirable that the acoustic impedance of the acoustic matching material 1300 is between the acoustic impedance of the subject 8 and the acoustic impedance of the acoustic wave reception elements 30. The acoustic matching material 1300 may be transparent to the illumination light 11 emitted from the illumination unit 1. The acoustic matching material 1300 may be a liquid. More specifically, the acoustic matching material 1300 may be water, castor oil, gel, or the like.

In the present embodiment, in step 400, the data reduction determination unit 15 determines an overlapping region where the region 60 overlaps the high sensitivity region 1500. Subsequently, the data reduction determination unit 15 determines intersections between the respective normal vectors of the reception planes of the acoustic wave reception elements 30-1 to 30-8 and the overlapping region between the region 60 and the high sensitivity region 1500 as illustrated in Fig. 8. Subsequently, based on the position information of the respective intersections and the position information of the respective acoustic wave reception elements 30, the data reduction determination unit 15 generates a storage control signal specifying whether the reception signal is to be stored or not and outputs the generated storage control signal to the signal data acquisition unit 7 as in the first embodiment. Then in step 500, based on the storage control signal, the signal data acquisition unit 7 generates reception signal data and stores it such that data of the reception signal of the photoacoustic wave generated in the overlapping region between the region 60 and the high sensitivity region 1500 is not stored.

By determining the period in which the amount of data is reduced as described above, it becomes possible to selectively store data of a reception signal obtained by receiving, with high sensitivity, a photoacoustic wave with a high generated sound pressure generated in a region in which the light fluence is high.

In the embodiments, the data reduction period may be set in units of distances or in units of time lengths. Alternatively, the data reduction period may be set in units of number of sampling clocks of the ADC, in units of number of system clocks, or in units of number of pieces of data. Any other method may be used to specify the data reduction period as long as it is possible to properly specify a corresponding region.

Although in the embodiments described above, it is assumed by way of example that there are eight acoustic wave reception elements. However, the number of acoustic wave reception elements is not limited to eight. Any number of acoustic wave reception elements may be used depending on the specifications of the apparatus.

The end timing of the data acquisition period may be set such that all acoustic wave reception elements have the same end timing or the end timing may be set separately for each acoustic wave reception element.

In a case where the end timing of the data acquisition period is set separately for each acoustic wave reception element, a determination may be performed based on the shape information of the subject to detect a region in which there is no subject on the directional axis of each acoustic wave reception element, and the end timing of the data acquisition period may be determined taking into account the detected region.

In the embodiments, the data reduction period does not necessarily need to be set separately for each acoustic wave reception element 30, but the plurality of acoustic wave reception elements 30 may be grouped and the data reduction period may be set on a group-by-group basis. For example, acoustic wave reception elements having nearly equal distances from a region in which the light fluence is higher than the threshold value may be grouped together, or acoustic wave reception elements located close to each other may be grouped together. This makes it possible to reduce the complexity in the setting process compared with the case where setting is performed separately for each acoustic wave reception element 30. Note that the manner of grouping acoustic wave reception elements may be varied depending on the measurement position. The manner of grouping acoustic wave reception elements may be varied from one measurement position to another, or may be varied from one group of measurement positions to another group. Even at the same measurement position, the manner of grouping acoustic wave reception elements may be varied when a manner of providing illumination light is changed.

The data reduction period may be different from one measurement position of the supporting unit 40 to another measurement position. The data reduction period may be set equally for a plurality of measurement positions.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

**Claims**

1. A photoacoustic apparatus comprising:

   a light source (20);
   a probe (2) including a reception element (30) configured to receive a photoacoustic wave generated by a subject (8) in response to being illuminated with light (11) from the light source (20) and output a time-series reception signal;
   a signal data acquisition unit (7) configured to generate reception signal data based on the time-series reception signal output from the reception element (30) and store the reception signal data;
   a light fluence acquisition unit (5) configured to acquire a light fluence at a position of interest in the subject; and
   an information acquisition unit (9) configured to acquire subject information based on the reception signal data stored in the signal data acquisition unit (7),
   wherein the signal data acquisition unit (7) generates the reception signal data, and stores the generated reception signal data, such that in a case where the light fluence at the position of interest is smaller than a threshold value, the amount of data is reduced for a part of the time-series reception signal that corresponds to the position of interest.

2. The photoacoustic apparatus according to claim 1, wherein in a case where the light fluence at the position of interest is smaller than the threshold value, the signal data acquisition unit does not store a part of the time-series reception signal that corresponds to the position of interest.

3. The photoacoustic apparatus according to claim 1, wherein
   the light fluence acquisition unit (5) acquires the light fluence at a plurality of positions of interest, and
   the signal data acquisition unit (7) samples the reception signal, corresponding to a position of interest where the light fluence acquired by the light fluence acquisition unit (5) is smaller than the threshold value, at a lower sampling frequency than a sampling frequency used to sample the reception signal corresponding to position of interest where the light fluence acquired by the light fluence acquisition unit is equal to or greater than the threshold value, thereby generating the reception signal data, and the signal data acquisition unit (7) stores the resultant reception signal data.

4. The photoacoustic apparatus according to one of claims 1 to 3, wherein
   the probe (2) includes a supporting unit (40) configured to support a plurality of reception elements (30-1 - 30-8),
   the supporting unit (40) supports the plurality of reception elements (30-1 - 30-8) such that directional axes of the respective reception elements converge in a particular region (1500), and
   the signal data acquisition unit (7) generates the reception signal data, and stores the generated reception signal data, such that in a case where the light fluence at the position of interest is smaller than the threshold value and the position of interest is not included in the particular region (1500), a part of the time-series reception signal that corresponds to the position of interest is subjected to the reduction in the amount of data.

5. The photoacoustic apparatus according to one of claims 1 to 3, wherein
   the probe (2) includes a supporting unit (40) with a shape based on a sphere, on a spherical surface of which a plurality of reception elements (30-1 - 30-8) are supported, and
   the signal data acquisition unit (7) generates the reception signal data, and stores the generated reception signal data, such that in a case where the light fluence at the position of interest is smaller than the threshold value and the position of interest is not at the center of curvature (P) of the shape of the supporting unit (40), a part of the time-series reception signal that corresponds to the position of interest is subjected to the reduction in the amount of data.

6. The photoacoustic apparatus according to claim 5, wherein the signal data acquisition unit (7) generates the reception signal data, and stores the generated reception signal data, such that in a case where the light fluence at the position of interest is smaller than the threshold value and the position of interest is not included in a high sensitivity region (1500) centered at the center of curvature (P) of the shape of the supporting unit (40), a part of the time-series reception signal that corresponds to the position of interest is subjected to the reduction in the amount of data.

7. The photoacoustic apparatus according to one of claims 1 to 6, wherein
   the light source (20) generates light at each of a plurality of timings,
   the reception element (30) receives a photoacoustic wave and outputs the time-series reception signal at each of the plurality of timings,
   the light fluence acquisition unit acquires the light fluence at the position of interest at each of the plurality of timings, and

the signal data acquisition unit generates the reception signal data, and stores the generated reception signal data, for each of the time-series reception signals respectively corresponding to the plurality of timings such that in a case where the light fluence at the position of interest is smaller than the threshold value, a part of the time-series reception signal that corresponds to the position of interest is subjected to the reduction in amount of data.

8. The photoacoustic apparatus according to claim 7, wherein the light source (20) generates light such that a condition of illuminating the subject with light is different among the plurality of timings.

9. The photoacoustic apparatus according to claim 7 or 8, further comprising
an optical system configured to direct the light emitted from the light source (20) such that the light reaches the subject, and
an optical system moving mechanism configured to move the optical system such that the position of the optical system is different from one to another of the plurality of timings.

10. The photoacoustic apparatus according to one of claims 7 to 9, further comprising a probe moving mechanism configured to move the probe (30) such that the position of the probe (30) is different from one to another of the plurality of timings.

11. The photoacoustic apparatus according to one of claims 1 to 10, wherein
the light fluence acquisition unit (5) acquires the light fluence at a plurality of positions of interest, and
the signal data acquisition unit (7) generates the reception signal data, and stores the generated reception signal data, at each of the plurality of timings such that in a case where the light fluence at the position of interest is smaller than the threshold value, a part of the time-series reception signal that corresponds to the position of interest is subjected to the reduction in amount of data.

12. The photoacoustic apparatus according to one of claims 1 to 11, further comprising
a control unit (3) configured to set a measurement parameter associated with an illumination condition,
wherein the light fluence acquisition unit (5) acquires the light fluence at the position of interest based on the measurement parameter set by the control unit (3).

13. The photoacoustic apparatus according to claim 12, further comprising
an input unit (4) configured to allow input of the measurement parameter,
wherein the control unit (3) sets the measurement parameter based on information associated with the measurement parameter output from the input unit.

14. The photoacoustic apparatus according to one of claims 1 to 13, wherein the information acquisition unit does not acquire the subject information at the position of interest when the light fluence at the position of interest is smaller than the threshold value.

15. A photoacoustic apparatus comprising:

a light source (20);
a probe (2) including a reception element (30) configured to receive a photoacoustic wave generated by a subject (8) in response to being illuminated with light (11) from the light source and output a time-series reception signal;
a light fluence acquisition unit (5) configured to acquire a light fluence distribution in the subject;
a data reduction determination unit (15) configured to make a determination based on the light fluence distribution acquired by the light fluence acquisition unit (5) as to a period in which the amount of data is to be reduced;
a signal data acquisition unit (7) configured to generate the reception signal data, and store the generated reception signal data, such that the amount of data is reduced for a part of the time-series reception signal that is output from the reception element during the period; and
an information acquisition unit (8) configured to acquire subject information based on the reception signal data stored in the signal data acquisition unit (7).

**Patentansprüche**

1. Photoakustische Vorrichtung, umfassend:

   eine Lichtquelle (20);
   eine Sonde (2), die ein Empfangselement (30) enthält, das konfiguriert ist, eine durch ein Subjekt (8) ansprechend auf dessen Beleuchtung mit Licht (11) aus der Lichtquelle (20) erzeugte photoakustische Welle zu empfangen und ein Zeitreihenempfangssignal auszugeben;
   eine Signaldatenerfassungseinheit (7), die konfiguriert ist zum Erzeugen von Empfangssignaldaten basierend auf dem durch das Empfangselement (30) ausgegebenen Zeitreihenempfangssignal und zum Speichern der Empfangssignaldaten;
   eine Leuchtstärkeerfassungseinheit (5), die konfiguriert ist zum Erfassen einer Leuchtstärke an einer Betrachtungsposition im Subjekt; und
   eine Informationserfassungseinheit (9), die konfiguriert ist zum Erfassen von Subjektinformation basierend auf den in der Signaldatenerfassungseinheit (7) gespeicherten Empfangssignaldaten,
   wobei die Signaldatenerfassungseinheit (7) die Empfangssignaldaten erzeugt und die erzeugten Empfangssignaldaten speichert, so dass, falls die Leuchtstärke an der Betrachtungsposition kleiner ist als ein Schwellenwert, die Datenmenge für einen Teil des der Betrachtungsposition entsprechenden Zeitreihenempfangssignals reduziert wird.

2. Photoakustische Vorrichtung nach Anspruch 1, wobei, falls die Leuchtstärke an der Betrachtungsposition kleiner ist als der Schwellenwert, die Signaldatenerfassungseinheit einen Teil des der Betrachtungsposition entsprechenden Zeitreihenempfangssignals nicht speichert.

3. Photoakustische Vorrichtung nach Anspruch 1, wobei
   die Leuchtstärkeerfassungseinheit (5) die Leuchtstärke an mehreren Betrachtungspositionen erfasst, und
   die Signaldatenerfassungseinheit (7) das Empfangssignal entsprechend einer Betrachtungsposition, wo die durch die Leuchtstärkeerfassungseinheit (5) erfasste Leuchtstärke kleiner ist als der Schwellenwert, mit einer geringeren Abtastfrequenz abtastet als einer Abtastfrequenz, die verwendet wird zum Abtasten des Empfangssignals entsprechend einer Betrachtungsposition, wo die durch die Leuchtstärkeerfassungseinheit erfasste Leuchtstärke gleich oder größer ist als der Schwellenwert, und dadurch die Empfangssignaldaten erzeugt werden und die Signaldatenerfassungseinheit (7) die resultierenden Empfangssignaldaten speichert.

4. Photoakustische Vorrichtung nach einem der Ansprüche 1 bis 3, wobei
   die Sonde (2) eine Trageeinheit (40) enthält, die konfiguriert ist zum Tragen mehrerer Empfangselemente (30-1 - 30-8),
   die Trageeinheit (40) die mehreren Empfangselemente (30-1 - 30-8) so trägt, dass Richtungsachsen der jeweiligen Empfangselemente in einem bestimmten Bereich (1500) zusammenlaufen, und
   die Signaldatenerfassungseinheit (7) die Empfangssignaldaten erzeugt und die erzeugten Empfangssignaldaten speichert, so dass, falls die Leuchtstärke an der Betrachtungsposition kleiner ist als der Schwellenwert und die Betrachtungsposition nicht im bestimmten Bereich (1500) enthalten ist, ein Teil des der Betrachtungsposition entsprechenden Zeitreihenempfangssignals der Datenmengenreduzierung unterzogen wird.

5. Photoakustische Vorrichtung nach einem der Ansprüche 1 bis 3, wobei
   die Sonde (2) eine Trageeinheit (40) mit einer kugelbasierten Form auf einer gewölbten Fläche enthält, von der mehrere Empfangselemente (30-1 - 30-8) getragen werden, und
   die Signaldatenerfassungseinheit (7) die Empfangssignaldaten erzeugt und die erzeugten Empfangssignaldaten speichert, so dass, falls die Leuchtstärke an der Betrachtungsposition kleiner ist als der Schwellenwert und die Betrachtungsposition nicht am Zentrum der Krümmung (P) der Form der Trageeinheit (40) liegt, ein Teil des der Betrachtungsposition entsprechenden Zeitreihenempfangssignals der Datenmengenreduzierung unterzogen wird.

6. Photoakustische Vorrichtung nach Anspruch 5, wobei die Signaldatenerfassungseinheit (7) die Empfangssignaldaten erzeugt und die erzeugten Empfangssignaldaten speichert, so dass, falls die Leuchtstärke an der Betrachtungsposition kleiner ist als der Schwellenwert und die Betrachtungsposition nicht in einem Bereich (1500) hoher Empfindlichkeit enthalten ist, welcher sich im Zentrum der Krümmung (P) der Form der Trageeinheit (40) befindet, ein Teil des der Betrachtungsposition entsprechenden Zeitreihenempfangssignals der Datenmengenreduzierung unterzogen wird.

**7.** Photoakustische Vorrichtung nach einem der Ansprüche 1 bis 6, wobei
die Lichtquelle (20) Licht zu jedem der mehreren Timings erzeugt,
das Empfangselement (30) eine photoakustische Welle empfängt und das Zeitreihenempfangssignal zu jedem der mehreren Timings ausgibt,
die Leuchtstärkeerfassungseinheit die Leuchtstärke an der Betrachtungsposition zu jedem der mehreren Timings erfasst, und
die Signaldatenerfassungseinheit die Empfangssignaldaten erzeugt und die erzeugten Empfangssignaldaten speichert für jedes der Zeitreihenempfangssignale, die jeweils den mehreren Timings entsprechen, so dass, falls die Leuchtstärke an der Betrachtungsposition kleiner ist als der Schwellenwert, ein Teil des der Betrachtungsposition entsprechenden Zeitreihenempfangssignals der Datenmengenreduzierung unterzogen wird.

**8.** Photoakustische Vorrichtung nach Anspruch 7, wobei die Lichtquelle (20) Licht erzeugt, so dass eine Beleuchtungsbedingung des Subjekts mit Licht unter den mehreren Timings unterschiedlich ist.

**9.** Photoakustische Vorrichtung nach Anspruch 7 oder 8, ferner umfassend ein optisches System, das konfiguriert ist, das von der Lichtquelle (20) emittierte Licht derart auszurichten, dass das Licht das Subjekt erreicht, und
ein Mechanismus zur Bewegung eines optischen Systems, der konfiguriert ist zum Bewegen des optischen Systems, so dass die Position des optischen Systems zu denjenigen zu den mehreren Timings unterschiedlich ist.

**10.** Photoakustische Vorrichtung nach einem der Ansprüche 7 bis 9, ferner umfassend einen Mechanisms zur Bewegung einer Sonde, der konfiguriert ist zum Bewegen der Sonde (30), so dass die Position der Sonde (30) zu denjenigen zu den mehreren Timings unterschiedlich ist.

**11.** Photoakustische Vorrichtung nach einem der Ansprüche 1 bis 10, wobei
die Leuchtstärkeerfassungseinheit (5) die Leuchtstärke an mehreren Betrachtungspositionen erfasst, und
die Signaldatenerfassungseinheit (7) die Empfangssignaldaten erzeugt und die erzeugten Empfangssignaldaten speichert, zu jedem der mehreren Timings, so dass, falls die Leuchtstärke an der Betrachtungsposition kleiner ist als der Schwellenwert, ein Teil des der Betrachtungsposition entsprechenden Zeitreihenempfangssignals der Datenmengenreduzierung unterzogen wird.

**12.** Photoakustische Vorrichtung nach einem der Ansprüche 1 bis 11, ferner umfassend
eine Steuereinheit (3), die konfiguriert ist zum Einstellen eines einer Beleuchtungsbedingung zugeordneten Messparameters,
wobei die Leuchtstärkeerfassungseinheit (5) die Leuchtstärke an der Betrachtungsposition basierend auf dem durch die Steuereinheit (3) eingestellten Messparameter erfasst.

**13.** Photoakustische Vorrichtung nach Anspruch 12, ferner umfassend
eine Eingabeeinheit (4), die konfiguriert ist zum Erlauben von Eingabe des Messparameters,
wobei die Steuereinheit (3) den Messparameter basierend auf der dem von der Eingabeeinheit ausgegebenen Messparameter zugeordneten Information einstellt.

**14.** Photoakustische Vorrichtung nach einem der Ansprüche 1 bis 13, wobei die Informationserfassungseinheit die Subjektinformation an der Betrachtungsposition nicht erfasst, wenn die Leuchtstärke an der Betrachtungsposition kleiner als der Schwellenwert ist.

**15.** Photoakustische Vorrichtung, umfassend:

eine Lichtquelle (20);
eine Sonde (2), die ein Empfangselement (30) enthält, das konfiguriert ist zum Empfangen einer durch ein Subjekt (8) ansprechend auf dessen Beleuchtung mit Licht (11) aus der Lichtquelle erzeugten photoakustischen Welle und zum Ausgeben eines Zeitreihenempfangssignals;
eine Leuchtstärkeerfassungseinheit (5), die konfiguriert ist zum Erfassen einer Leuchtstärkeverteilung im Subjekt;
eine Datenreduzierbestimmungseinheit (15), die konfiguriert ist zum Bestimmen basierend auf der durch die Leuchtstärkeerfassungseinheit (5) erfassten Leuchtstärkeverteilung betreffend einen Zeitraum, in dem die Datenmenge zu reduzieren ist;
eine Signaldatenerfassungseinheit (7), die konfiguriert ist zum Erzeugen der Empfangssignaldaten und zum Speichern der erzeugten Empfangssignaldaten, so dass die Datenmenge für einen Teil des Zeitreihenempfangssignals reduziert wird, das während des Zeitraums vom Empfangselement ausgegeben wird; und eine Informationserfassungseinheit (8), die konfiguriert ist zum Erfassen von Subjektinformation basierend auf den in der Signaldatenerfassungseinheit (7) gespeicherten Empfangssignaldaten.

**Revendications**

1. Appareil photoacoustique comprenant :

   une source lumineuse (20) ;
   une sonde (2) comportant un élément de réception (30) configuré pour recevoir une onde photoacoustique générée par un sujet (8) en réponse au fait d'être éclairé par de la lumière (11) en provenance de la source lumineuse (20) et fournir en sortie un signal de réception de série temporelle ;
   une unité d'acquisition de données de signaux (7) configurée pour générer des données de signaux de réception sur la base du signal de réception de série temporelle fourni en sortie par l'élément de réception (30) et stocker les données de signaux de réception ;
   une unité d'acquisition de fluence de la lumière (5) configurée pour acquérir une fluence de la lumière à une position d'intérêt dans le sujet ; et
   une unité d'acquisition d'informations (9) configurée pour acquérir des informations de sujet sur la base des données de signaux de réception stockées dans l'unité d'acquisition de données de signaux (7),
   dans lequel l'unité d'acquisition de données de signaux (7) génère les données de signaux de réception, et stocke les données de signaux de réception générées, de façon que, dans un cas où la fluence de la lumière à la position d'intérêt est inférieure à une valeur de seuil, la quantité de données soit réduite pour un partie du signal de réception de série temporelle qui correspond à la position d'intérêt.

2. Appareil photoacoustique selon la revendication 1, dans lequel, dans un cas où la fluence de la lumière à la position d'intérêt est inférieure à la valeur de seuil, l'unité d'acquisition de données de signaux ne stocke pas une partie du signal de réception de série temporelle qui correspond à la position d'intérêt.

3. Appareil photoacoustique selon la revendication 1, dans lequel
   l'unité d'acquisition de fluence de la lumière (5) acquiert la fluence de la lumière en une pluralité de positions d'intérêt, et
   l'unité d'acquisition de données de signaux (7) échantillonne le signal de réception, correspondant à une position d'intérêt où la fluence de la lumière acquise par l'unité d'acquisition de fluence de la lumière (5) est inférieure à la valeur de seuil, à une fréquence d'échantillonnage inférieure à une fréquence d'échantillonnage utilisée pour échantillonner le signal de réception correspondant à la position d'intérêt où la fluence de la lumière acquise par l'unité d'acquisition de fluence de la lumière est supérieure ou égale à la valeur de seuil, pour ainsi générer les données de signaux de réception, et l'unité d'acquisition de données de signaux (7) stocke la résultante des données de signaux de réception.

4. Appareil photoacoustique selon l'une des revendications 1 à 3, dans lequel
   la sonde (2) comprend une unité de support (40) configurée pour supporter une pluralité d'éléments de réception (30-1 - 30-8),
   l'unité de support (40) supporte la pluralité d'éléments de réception (30-1 - 30-8) de façon que les axes directionnels des éléments de réception respectifs convergent dans une région particulière (1500), et
   l'unité d'acquisition de données de signaux (7) génère les données de signaux de réception, et stocke les données de signaux de réception générées, de façon que, dans un cas où la fluence de la lumière à la position d'intérêt est inférieure à la valeur de seuil et où la position d'intérêt n'est pas contenue dans la région particulière (1500), une partie du signal de réception de série temporelle qui correspond à la position d'intérêt soit soumise à une réduction de la quantité de données.

5. Appareil photoacoustique selon l'une des revendications 1 à 3, dans lequel
   la sonde (2) comprend une unité de support (40) ayant une forme à base de sphère, sur une surface sphérique par laquelle sont supportés une pluralité d'éléments de réception (30-1 - 30-8), et
   l'unité d'acquisition de données de signaux (7) génère les données de signaux de réception, et stocke les données de signaux de réception générées, de façon que, dans un cas où la fluence de la lumière à la position d'intérêt est inférieure à la valeur de seuil et où la position d'intérêt n'est pas au centre de courbure (P) de la forme de l'unité de support (40), une partie du signal réception de série temporelle qui correspond à la position d'intérêt soit soumise à une réduction de la quantité de données.

6. Appareil photoacoustique selon la revendication 5, dans lequel l'unité d'acquisition de données de signaux (7) génère les données de signaux de réception, et stocke les données de signaux de réception générées, de façon que, dans un cas où la fluence de la lumière à la position d'intérêt est inférieure à la valeur de seuil et où la position d'intérêt n'est pas contenue dans une région de haute sensibilité (1500) centrée sur le centre de courbure (P) de la forme de l'unité de support (40), une partie du signal de réception de série temporelle qui correspond à la position d'intérêt soit soumise à une réduction de la quantité de données.

7. Appareil photoacoustique selon l'une des revendications 1 à 6, dans lequel
la source lumineuse (20) génère la lumière à chacun d'une pluralité d'instants,
l'élément de réception (30) reçoit une onde photoacoustique et fournit en sortie le signal de réception de série temporelle à chacun de la pluralité d'instants,
l'unité d'acquisition de fluence de la lumière acquiert la fluence de la lumière à la position d'intérêt à chacun de la pluralité d'instants, et
l'unité d'acquisition de données de signaux génère les données de signaux de réception, et stocke les données de signaux de réception générées, pour chacun des signaux de réception de série temporelle respectifs correspondant à la pluralité d'instants de façon que, dans un cas où la fluence de la lumière à la position d'intérêt est inférieure à la valeur de seuil, une partie du signal de réception de série temporelle qui correspond à la position d'intérêt soit soumise à une réduction de la quantité de données.

8. Appareil photoacoustique selon la revendication 7, dans lequel la source lumineuse (20) génère la lumière de façon qu'une condition d'éclairage du sujet par la lumière soit différente parmi la pluralité d'instants.

9. Appareil photoacoustique selon la revendication 7 ou 8, comprenant en outre
un système optique configuré pour diriger la lumière émise par la source lumineuse (20) de façon que la lumière atteigne le sujet, et
un mécanisme de déplacement de système optique configuré pour déplacer le système optique de façon que la position du système optique soit différente de l'un à l'autre de la pluralité d'instants.

10. Appareil photoacoustique selon l'une des revendications 7 à 9, comprenant en outre un mécanisme de déplacement de sonde configuré pour déplacer la sonde (30) de façon que la position de la sonde (30) soit différente de l'un à l'autre de la pluralité d'instants.

11. Appareil photoacoustique selon l'une des revendications 1 à 10, dans lequel
l'unité d'acquisition de fluence de la lumière (5) acquiert la fluence de la lumière en une pluralité de positions d'intérêt, et
l'unité d'acquisition de données de signaux (7) génère les données de signaux de réception, et stocke les données de signaux de réception générées à chacun de la pluralité d'instants de façon que, dans un cas où la fluence de la lumière à la position d'intérêt est inférieure à la valeur de seuil, une partie du signal de réception de série temporelle qui correspond à la position d'intérêt soit soumise à une réduction de la quantité de données.

12. Appareil photoacoustique selon l'une des revendications 1 à 11, comprenant en outre
une unité de commande (3) configurée pour définir un paramètre de mesure associé à une condition d'éclairement, dans lequel l'unité d'acquisition de fluence de la lumière (5) acquiert la fluence de la lumière à la position d'intérêt sur la base du paramètre de mesure réglé par l'unité de commande (3).

13. Appareil photoacoustique selon la revendication 12, comprenant en outre
une unité d'entrée (4) configurée pour permettre la fourniture en entrée du paramètre de mesure,
dans lequel l'unité de commande (3) règle le paramètre de mesure sur la base d'informations associées au paramètre de mesure fourni en sortie par l'unité d'entrée.

14. Appareil photoacoustique selon l'une des revendications 1 à 13, dans lequel l'unité d'acquisition d'informations n'acquiert pas les informations de sujet à la position d'intérêt lorsque la fluence de la lumière à la position d'intérêt est inférieure à la valeur de seuil.

15. Appareil photoacoustique comprenant :

une source lumineuse (20) ;

une sonde (2) comportant un élément de réception (30) configuré pour recevoir une onde photoacoustique générée par un sujet (8) en réponse au fait d'être éclairé par de la lumière (11) en provenance de la source lumineuse et fournir en sortie un signal de réception de série temporelle ;

une unité d'acquisition de fluence de la lumière (5) configurée pour acquérir une distribution de la fluence de la lumière dans le sujet ;

une unité de détermination de réduction de données (15) configurée pour effectuer une détermination sur la base de la distribution de la fluence de la lumière acquise par l'unité d'acquisition de fluence de la lumière (5) en ce qui concerne une période lors de laquelle la quantité de données doit être réduite ;

une unité d'acquisition de données de signaux (7) configurée pour générer les données de signaux de réception, et stocker les données de signaux de réception générées, de façon que la quantité de données soit réduite pour une partie du signal de réception de série temporelle qui est fournie en sortie par l'élément de réception pendant la période ; et

une unité d'acquisition d'informations (8) configurée pour acquérir des informations de sujet sur la base des données de signaux de réception stockées dans l'unité d'acquisition de données de signaux (7).

FIG. 1

# FIG. 2

```
                    ┌─────────────────────────┐
                    │    START MEASUREMENT    │
                    └─────────────────────────┘
                                 │
                                 ▼
        ┌───────────────────────────────────────────────┐
        │          SET MEASUREMENT PARAMETER            │─────  100
        └───────────────────────────────────────────────┘
                                 │
                                 ▼
        ┌───────────────────────────────────────────────┐
        │      ESTIMATE DISTRIBUTION OF LIGHT FLUENCE    │─────  200
        │    ACROSS POSITIONS OF INTEREST IN SUBJECT     │
        └───────────────────────────────────────────────┘
                                 │
                                 ▼
        ┌───────────────────────────────────────────────┐
        │      DETERMINE POSITION OF INTEREST AT WHICH   │─────  300
        │   LIGHT FLUENCE IS SMALLER THAN THRESHOLD VALUE│
        └───────────────────────────────────────────────┘
                                 │
                                 ▼
        ┌───────────────────────────────────────────────┐
        │    DETERMINE PERIOD IN WHICH DATA IS TO BE REDUCED │─────  400
        └───────────────────────────────────────────────┘
                                 │
                                 ▼
        ┌───────────────────────────────────────────────┐
        │           ILLUMINATE SUBJECT WITH LIGHT       │─────  500
        └───────────────────────────────────────────────┘
                                 │
                                 ▼
        ┌───────────────────────────────────────────────┐
        │           RECEIVE PHOTOACOUSTIC WAVE          │─────  600
        └───────────────────────────────────────────────┘
                                 │
                                 ▼
        ┌───────────────────────────────────────────────┐
        │           STORE RECEIVED SIGNAL DATA          │─────  700
        └───────────────────────────────────────────────┘
                                 │
                                 ▼
        ┌───────────────────────────────────────────────┐
        │         ACQUIRE SUBJECT INFORMATION AT        │─────  800
        │     EACH POSITION OF INTEREST IN SUBJECT      │
        └───────────────────────────────────────────────┘
                                 │
                                 ▼
        ┌───────────────────────────────────────────────┐
        │      DISPLAY IMAGE BASED ON SUBJECT INFORMATION│─────  900
        └───────────────────────────────────────────────┘
                                 │
                                 ▼
                    ┌─────────────────────────┐
                    │    END MEASUREMENT      │
                    └─────────────────────────┘
```

## FIG. 3A

## FIG. 3B

## FIG. 3C

## FIG. 3D

FIG. 4

DATA REDUCTION
DETERMINATION UNIT ~15

WRITE ENABLE [1]-[8]
READ ENABLE [1]-[8]

FIFO
CONTROL UNIT ~712

SYSTEM CLK ~713

717-1
30-1   ADC   [1]   ~716-1   FIFO
717-2
30-2   ADC   [2]   ~716-2   FIFO
717-3
30-3   ADC   [3]   ~716-3   FIFO
717-4
30-4   ADC   [4]   ~716-4   FIFO
717-5
30-5   ADC   [5]   ~716-5   FIFO
717-6
30-6   ADC   [6]   ~716-6   FIFO
717-7
30-7   ADC   [7]   ~716-7   FIFO
717-8
30-8   ADC   [8]   ~716-8   FIFO

714

718
DRAM

7

9

722
DRAM

721
GPU

EP 2 946 725 B1

# FIG. 5

EP 2 946 725 B1

# FIG. 6A

# FIG. 6B

# FIG.6C

# FIG. 7

# FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5713356 A

- WO 2011039979 A1

**Non-patent literature cited in the description**

- **LIHONG V. WANG SONG HU.** Photoacoustic Tomography: In Vivo Imaging From Organelles to Organs. *Science,* 2012, vol. 335, 1458